# EUROPEAN PATENT APPLICATION

(11) **EP 4 546 362 A1**
(43) Date of publication of application: **30.04.2025**
(21) Application number: 24207663.6
(22) Date of filing: 18.10.2024
(51) Int. Cl.: G16H 20/40, G16H 40/63, G16H 40/67

(54) **CARE ENVIRONMENT VIDEO/AUDIO MONITORING SYSTEM**

(30) Priority: 23.10.2023 US 202363545311 P
(71) Applicant: Draeger Medical Systems, Inc., Andover, MA 01810 (US)
(72) Inventor: THACKER, Christopher P, Telford, 18969 (US)
(74) Representative: Haseltine Lake Kempner LLP

(57) **Abstract**

The present disclosure provides a multi-patient monitoring capability to medical thermoregulation machines for neonatal patients. A centralized monitoring station for monitoring multiple neonatal patients provides visual and auditory observations, as well as other sensed conditions, to a caregiver. This capability permits rapid observation, assessment, and reaction to neonatal patient conditions responsive to adverse developments. The capability may also permit lower head counts among nursing and other staff while, at the same time, improving patient care.

## Description

### RELATED APPLICATIONS

Not applicable.

### TECHNICAL FIELD

The present disclosure relates generally to the field of medical monitoring of a patient and, more particularly, to monitoring a groups of neonate's conditions where each neonate is in an individual warming therapy device.

### BACKGROUND

This section of this document introduces information about and/or from the art that may provide context for or be related to the subject matter described herein and/or claimed below. It provides background information to facilitate a better understanding of the various aspects of the present invention. This is a discussion of "related" art. That such art is related in no way implies that it is also "prior" art. The related art may or may not be prior art. The discussion in this section of this document is to be read in this light, and not as admissions of prior art.

Patient observation is an important aspect of medical care for neonatal patients, particularly in the first few days after birth. This remains true for unfortunate neonatal patients whose condition results in admission to a Neonatal intensive Care Unit ("NICU"), even after weeks or months of care. Accordingly, the art continually seeks better and more effective techniques for monitoring neonatal patients. This quest includes not only new and better techniques, but also equipment for implementing such techniques.

### SUMMARY

The present disclosure provides a multi-patient monitoring capability to medical thermoregulation machines for neonatal patients. Visual and auditory observations are frequently the first indicators to the condition of a neonatal patient. A centralized monitoring station for monitoring multiple neonatal patients provides visual and auditory observations, as well as other sensed conditions, to a caregiver. This capability permits rapid observation, assessment, and reaction to neonatal patient conditions responsive to adverse developments. The capability may also permit lower head counts among nursing and other staff while, at the same time, improving patient care.

In a first aspect, a neonatal patient monitoring system comprises a central monitoring station, including a display device and a central monitoring controller. The central monitoring controller is programmed to: receive a plurality of transmitted patient care data from each of a plurality of warming therapy devices; in a first state, concurrently provide a respective dormant patient display for each warming therapy device on the display device; and in a second state, provide on the display device an active patient display for a particular one of the warming therapy devices, the active patient display being larger than the dormant patient display.

In a second aspect, a method for neonatal monitoring comprises: receiving a plurality of transmitted patient care data from each of a plurality of warming therapy devices; in a first state, concurrently providing a respective dormant patient display for each warming therapy device on the display device; and in a second state, providing on the display device an active patient display for a particular one of the warming therapy devices, the active patient display being larger than the dormant patient display.

In a third aspect, a neonatal patient monitoring system comprises a plurality of warming therapy devices and a central monitoring station. Each warming therapy device defines a respective care environment and includes a plurality of sensors, and a warming therapy device controller. The plurality of sensors capture patient care data when the warming therapy device is in use and includes a camera, a microphone, and a galvanic skin response sensor. The camera positioned in the respective care environment and directed on a patient location to capture video imagery of the neonatal patient; the microphone positioned in the care environment to capture vocalizations of the neonatal patient; and the galvanic skin response sensor to capture the galvanic electrodermal response of the neonatal patient. The warming therapy device controller programmed to: transmit the captured patient care data; analyze the captured patient care data for alarm conditions; and if an alarm condition is detected, transmit an alarm signal. The central monitoring station includes: a display device, and a central monitoring controller. The central monitoring station controller is programmed to: receive the transmitted patient care data and transmitted alarm signals from each warming therapy device; in a first state, concurrently provide a respective dormant patient display for each warming therapy device on the display device; and in a second state, provide on the display device an active patient display for a particular one of the warming therapy devices, the active patient display being larger than the dormant patient display.

In a fourth aspect, a method for neonatal patient monitoring comprises: for each of a plurality of warming therapy devices, sensing a plurality of patient care data from within a care environment for a neonatal patient, the patient care data including: video imagery of the neonatal patient; audio capture of vocalizations of the neonatal patient; and a galvanic skin response of the neonatal patient; receiving the sensed patient care data at a warming therapy device, the warming therapy device programmed to: transmit the received patient care data; analyze the received patient care data for alarm conditions; and if an alarm condition is detected, transmit an alarm signal; and receiving at a centralized monitoring station the transmitted patient care data and the transmitted alarm signals from each warming therapy device, the centralized monitoring station: in a first state, concurrently provide a respective dormant patient display for each warming therapy device on the display device; and in a second state, provide on the display device an active patient display for a particular, the active patient display being larger than the dormant patient display.

In a fifth aspect, a neonatal patient monitoring system comprises a plurality of warming therapy devices and a central monitoring station. Each warming therapy device defining a respective care environment and includes a plurality of sensors and a warming therapy device controller. The plurality of sensors capture patient care data when the warming therapy device is in use, the patient care data including video imagery of a neonatal patient and audio data of the neonatal patient's vocalizations. The warming therapy device controller programmed to: transmit the captured patient care data; analyze the captured patient care data for alarm conditions; and if an alarm condition is detected, transmit an alarm signal. The central monitoring station includes a display device, and a central monitoring controller. The central monitoring station controller is programmed to: receive the transmitted patient care data and transmitted alarm signals from each warming therapy device; in a first state, concurrently provide a respective dormant patient display for each warming therapy device on the display device; and in a second state, automatically provide on the display device an active patient display for a particular warming therapy device triggered by receiving an alarm signal transmitted from the particular warming therapy device. The active patient display is larger than the dormant patient display and includes: the video imagery received from each warming therapy device; a button by which a caregiver may selectively play the neonatal patient's vocalizations; an identity of the alarm condition; and received patient care data associated with the alarm condition.

In a sixth aspect, a method for neonatal monitoring, comprises: for each of a plurality of warming therapy devices, sensing a plurality of patient care data from within a care environment for a neonatal patient, the patient care data including: video imagery of the neonatal patient; and audio capture of vocalizations of the neonatal patient; and receiving the sensed patient care data at a warming therapy device, the warming therapy device programmed to: transmit the received patient care data; analyze the received patient care data for alarm conditions; and if an alarm condition is detected, transmit an alarm signal; and receiving at a centralized monitoring station the transmitted patient care data and the transmitted alarm signals from each warming therapy device, the centralized transmitting station: in a first state, concurrently provide a respective dormant patient display for each warming therapy device on the display device; and in a second state, automatically provide on the display device an active patient display for a particular warming therapy device triggered by receiving an alarm signal transmitted from the particular warming therapy device, the active patient display being larger than the dormant patient display and including: the video imagery received from each warming therapy device; a button by which a caregiver may selectively play the neonatal patient's vocalizations; an identity of the alarm condition; and received patient care data associated with the alarm condition.

In a seventh aspect, a neonatal patient monitoring system is substantially as is shown and described herein.

In an eighth aspect, a method for neonatal monitoring is substantially as is shown and described.

The above presents a simplified summary of the invention as claimed below in order to provide a basic understanding of some aspects of the invention. This summary is not an exhaustive overview of the invention. It is not intended to identify key or critical elements of the invention or to delineate the scope of the invention. Its sole purpose is to present some concepts in a simplified form as a prelude to the more detailed description that is discussed later.

### BRIEF DESCRIPTION OF THE DRAWINGS

In the drawings, like reference numbers generally indicate identical, functionally similar, and/or structurally similar elements.
FIG. 1 conceptually illustrates a patient monitoring system in accordance with one or more embodiments.
FIG. 2 is an enlarged, detailed, perspective view of a representative warming therapy device first shown in FIG. 1.
FIG. 3 depicts a camera and a microphone as may be positioned in the respective care environment and directed on a patient location to capture video imagery of the neonatal patient and to capture vocalizations of the neonatal patient, respectively.
FIG. 4A and FIG. 4B depict the capture of video imagery of the neonatal patient and the capture of the vocalizations of the neonatal patient.
FIG. 5 schematically illustrates a plurality of sensors and the warming therapy device of FIG. 1 and FIG. 2.
FIG. 6 schematically illustrates a central monitoring station first shown in FIG. 1.
FIG. 7 illustrates one particular embodiment of the patient monitoring system of FIG. 1 highlighting certain aspects thereof to further an understanding of the invention.
FIG. 8 illustrates a method for neonatal monitoring in accordance with one or more embodiments.
FIG. 9 depict one particular embodiment of the display in a first state of operation for the central monitoring station.
FIG. 10A-FIG. 10D depict embodiments of the display in a second state of operation for the central monitoring station.
FIG. 11 illustrates one particular embodiment in which the active patient display is selected by a caregiver.
FIG. 12 illustrates one particular embodiment in which a detected alarm condition prompts an automatic display of the active patient display.

While the disclosed subject matter is susceptible to various modifications and alternative forms, the drawings illustrate specific implementations described in detail by way of example. It should be understood, however, that the description herein of specific examples is not intended to limit that which is claimed to the particular forms disclosed, but on the contrary, the intention is to cover all modifications, equivalents, and alternatives falling within the spirit and scope of the appended claims.

### DETAILED DESCRIPTION

The present disclosure provides a multi-patient monitoring capability to medical thermoregulation machines for neonatal patients. A plurality of warming therapy devices integrate a camera and a microphone inside the controlled environment of the warming therapy device for patient monitoring in a neonatal care setting. The audio and visual information from each warming therapy device is forwarded to a centralized monitoring station where a nurse or other caregiver can then visually see and selectively choose to hear any one of multiple neonatal patients concurrently. The monitoring display may display, for example, information such as patient live video, patient alarm status (especially on occurrence), patient identifying information (such as name, identification number, location), patient vital signs (e.g., temperature, blood oxygen saturation, etc.), environmental conditions (e.g., temperature, humidity, etc.), audio of neonatal patient vocalizations, and equipment conditions (e.g., canopy open, door latch status, etc.).

Additional sensors may be included to provide information indicative of the neonatal patients' levels of distress. For example, movement-whether absent or excessive-can indicate that a neonatal patient is in distress, and so a motion sensor may be employed. Similarly, galvanic, or electrodermal, skin resistance can indicate stress. More particularly, stress can subliminally or subconsciously increase sweat levels, which can increase skin conductivity (and, concomitantly, reduce skin resistance).

The sensed data can be acquired and transmitted to the central monitoring station. The central monitoring station, in a first state, displays a respective dormant patient display for each warming therapy device on a display device. Each "dormant patient display" includes the video feed from the warming therapy device and a button by which the caregiver can selectively play the accompanying audio feed. The dormant patient display may also include additional information such as environmental conditions in the care environment and/or patient care parameters.

The display of the central monitoring station can also, in a second state, automatically provide an "active patient display", the second state being triggered by receiving an alarm signal transmitted from the particular warming therapy device. The active patient display is larger than the dormant patient display, and may occupy the entire display device, in order to provide superior detail of the neonatal patient to the caregiver. The active patient display may include the video feed from the respective warming therapy device and the button for selectively playing the audio feed. The active patient display may also include additional information such as the identity of the alarm condition and received patient care data associated with the alarm condition.

In some embodiments, the active patient display may also be provided in a third state of the centralized monitoring station upon selective activation by a caregiver. Thus, a caregiver viewing the dormant patient display may choose to obtain a better view of a particular neonatal patient. The caregiver can then select the particular warming therapy device to obtain the active patient display. The active patient display may be the same or different in the second state and the third state, particularly in the display of patient care data. For example, there may not be an alarm such that the alarm identification is not available for display.

Turning now to the drawings, illustrative examples of the subject matter claimed below are disclosed. In the interest of clarity, not all features of an actual implementation are described for every example in this specification. It will be appreciated that in the development of any such actual implementation, numerous implementation-specific decisions may be made to achieve the developers' specific goals, such as compliance with system-related and business-related constraints, which will vary from one implementation to another. Moreover, it will be appreciated that such a development effort, even if complex and time-consuming, would be a routine undertaking for those of ordinary skill in the art having the benefit of this disclosure.

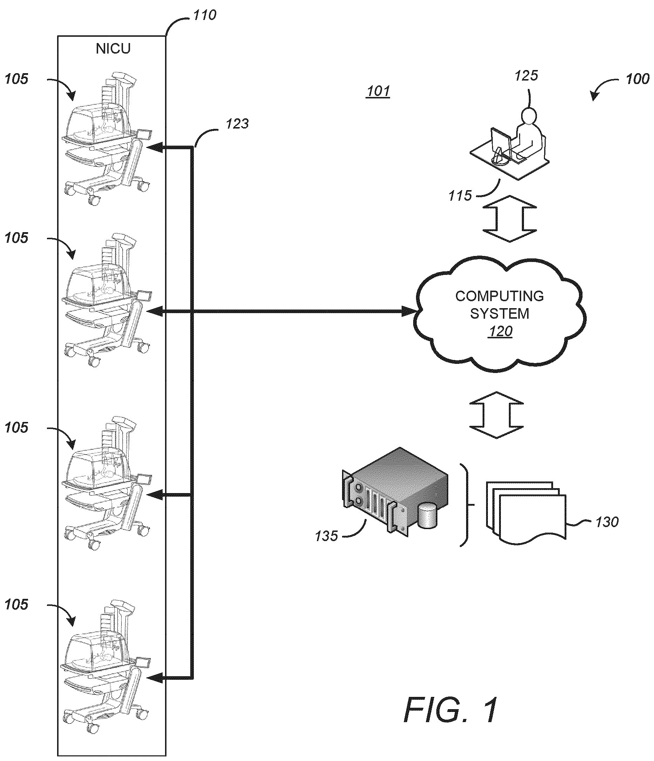

FIG. 1 conceptually illustrates a patient monitoring system 100 in accordance with one or more embodiments. The patient monitoring system 100 is implemented in a medical facility 101 housing a plurality of warming therapy devices 105 in a neonatal intensive care unit ("NICU") 110. The warming therapy devices 105 communicate with a centralized monitoring station 115 over a computing system 120 over a plurality of connections 123 that may be wired or wireless, as discussed further below. The connections 123 may or may not be considered a part of the computing system 120 depending upon the embodiment. Those skilled in the art having the benefit of this disclosure will appreciate that this is but one embodiment and that many variations may be realized. For example, it is not necessary that the warming therapy devices 105 be all housed together or even in a NICU. Still other variations will become apparent to those skilled in the art having the benefit of this disclosure.

The warming therapy devices 105 may be any kind of neonatal or infant care apparatus that provides a care environment, or a hybrid care environment, or an open warming device. for a neonatal or infant patient. For example, the warming therapy devices 105 may be warmers, incuwarmers, closed care apparatuses and hybrid-warming devices, *etc.* These examples are neither exclusive nor exhaustive and other types of warming therapy devices may be included. In the illustrated embodiments, the warming therapy devices are incubators, but the subject matter claimed below is not so limited. Again, any neonatal or infant care apparatus that provides a care environment for a neonatal or infant patient may be used in various embodiments.

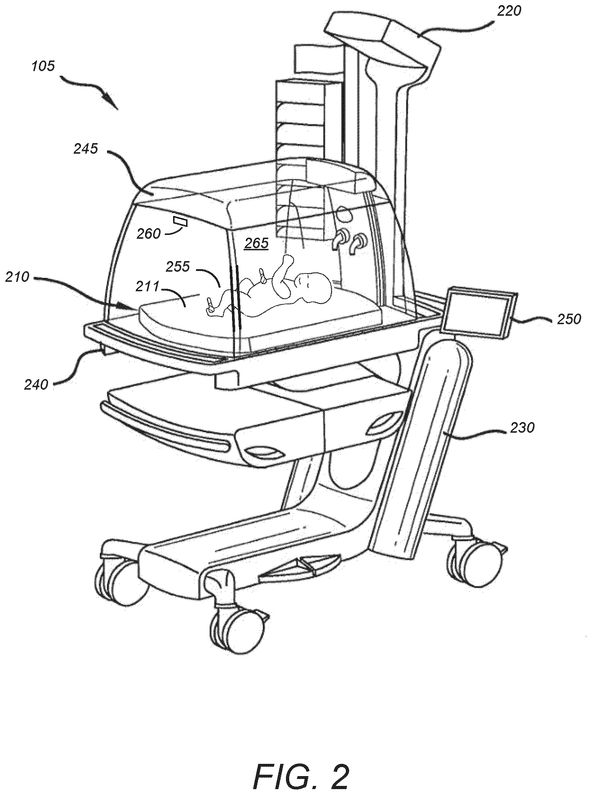

FIG. 2 is enlarged, detailed, perspective view of a representative warming therapy device 105. The warming therapy device 105 includes a radiant heater head 220, a patient support assembly 230, and a mattress tray assembly 240. The mattress tray assembly 240 also includes a hood 245, and a mattress tray 210, with a mattress 211 disposed therein. Additionally, the warming therapy device 105 may include at least one computer monitor 250 for displaying various information associated with a neonatal patient 255 disposed in the warming therapy device 105.

The computer monitor 250 may be coupled to the patient support assembly 230 of the warming therapy device 105 in either a wired or wireless manner. If the computer monitor 250 is wirelessly coupled to the patient support assembly 230, a caregiver (not shown) can remove the computer monitor 250 from the patient support assembly 230 and move around with it within a wireless coverage zone not indicated. In such an embodiment, the monitor 250 would preferably include a rechargeable internal battery (not shown) which would provide power during wireless use.

Similarly, the patient support assembly 230 would preferably include a charging unit for the internal battery, to charge the monitor 250 when it is stationed on the patient support assembly 230. Although the computer monitor 250 is shown as being coupled to the patient support assembly 230 in FIG. 2, those of ordinary skill in the art having the benefit of this disclosure that this may vary in other embodiments. The computer monitor 250 may be coupled to any suitable portion of the warming therapy device 105, or may be coupled to a separate unit entirely, in other embodiments.

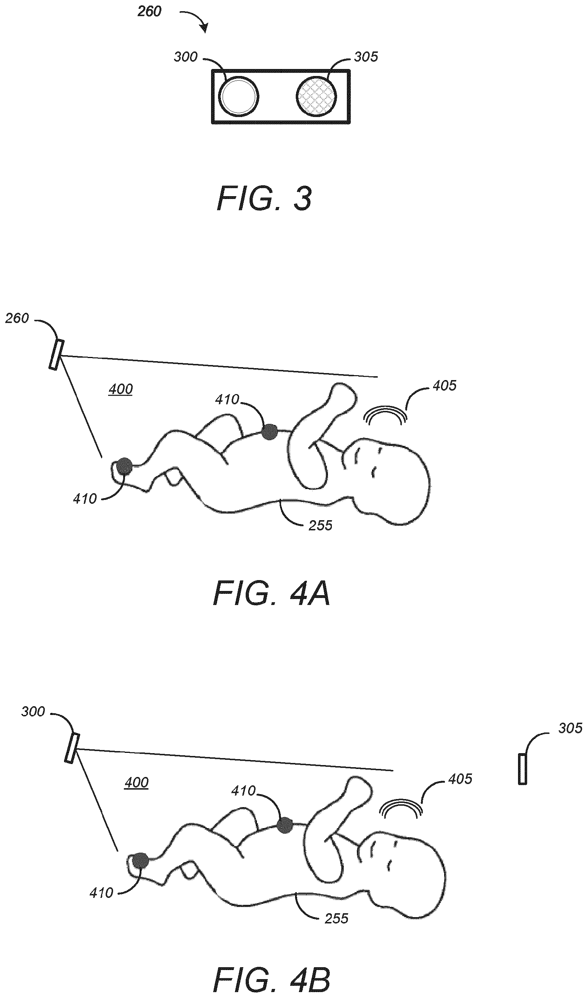

The warming therapy device 105 also includes an audio/visual ("A/V") device 260. As better shown in FIG. 3, the audio/visual device 260 includes a camera 300 and a microphone 305 integrated in a single device. As shown in FIG. 2, the audio/visual device 260 is positioned inside the care environment 265 defined by the mattress tray assembly 240. Thus, camera 300 positioned in the respective care environment 265 and directed on a patient location to capture video imagery of the neonatal patient 255 as indicated by the field of view 400. The microphone 305 is likewise positioned in the care environment 265 to capture vocalizations 405 of the neonatal patient 255.

However, there is no requirement that the camera 300 and microphone 305 be integrated in a single device or closely located within the care environment. As shown in FIG. 4B, they may be separated in some embodiments. This has the advantage of permitting separate positioning of the camera 300 and microphone 305 to optimize, or at least improve, their data acquisition. For example, in FIG. 4B, the microphone 305 is shown positioned near the head of the neonatal patient 255 to better pick up the vocalizations 405.

In some embodiments, the camera 300 and the microphone 305 may be integrated into, and be a part of, the warming therapy devices 105. Accordingly, in FIG. 4A, the camera 300 and the microphone 305 may be a device separate from the warming therapy device 105 or may be integrated into the warming therapy device 105. In FIG. 4B, the camera 300 and the microphone 305 may be individual sensors separately positioned, or may be individual devices separately positioned and integrated into the warming therapy device 105. In still other embodiments, one of the camera 300 and the microphone 305 may be a separate device while the other is integrated into the warming therapy device 105.

Also as shown in FIG. 4A and FIG. 4B, this particular embodiment includes skin galvanic, or electrodermal, response sensors 410. The skin galvanic response sensors 410 may be wired or wireless. More likely, they will be wired with the sensors 410 terminating electrical leads that are not shown in FIG. 4A and FIG. 4B.

In addition to the camera 300 and the microphone 305, the warming therapy devices 105 will include other sensors not yet shown. These other sensors may include, for example, one or more patient condition sensors to sense patient condition parameters. The patient condition sensors may sense blood oxygen saturation ("SpO2"), non-invasive blood pressure ("NIBP"), end-tidal carbon dioxide ("etCO2"), or body temperature, or skin temperature, or skin galvanic response, or motion, etc., or a combination thereof. For another example, these other sensors may include one or more environmental condition sensors to sense environmental parameters of the care environment. The environmental condition sensors may sense humidity, oxygen levels, or air temperature, etc., or a combination thereof. For another example, these other sensors may include one or more warming therapy device condition sensors to sense operational state of the warming therapy device 105. The warming therapy device condition sensors may sense power to the warming therapy device, warming therapy device on, warming therapy device closed, etc., or combinations thereof.

The examples listed in the immediately preceding paragraph are non-limiting examples. Those skilled in the art having the benefit of this disclosure will appreciate that the types of sensors and the parameters sensed may be found in practically any permutation depending on implementation-specific details. Such artisans will also appreciate from this disclosure that still types of sensors and parameters other than those listed may be found in other embodiments.

In general, it is contemplated by the present disclosure that warming therapy device 105 includes electronic components and/or electronic computing devices operable to receive, transmit, process, store, and/or manage patient data and information associated performing the functions of the system as described herein, which encompasses any suitable processing device adapted to perform computing tasks consistent with the execution of computer-readable instructions stored in a memory or a computer-readable recording medium.

Further, any, all, or some of the computing devices in warming therapy device 105 may be adapted to execute any operating system, including Linux^{®}, UNIX^{®}, Windows Server^{®}, *etc.,* as well as virtual machines adapted to virtualize execution of a particular operating system, including customized and proprietary operating systems. Warming therapy device 105 may be further equipped with components to facilitate communication with other computing devices over one or more network connections, which may include connections to local and wide area networks, wireless and wired networks, public and private networks, and any other communication network enabling communication in the system.

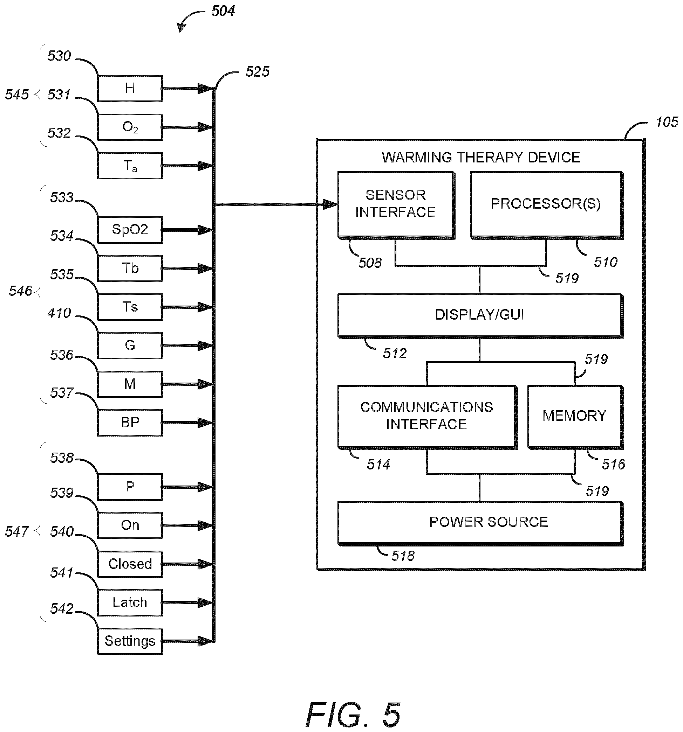

FIG. 5 schematically illustrates the electronics organization of one particular implementation of the warming therapy device 105. As shown in FIG. 5, warming therapy device 105 may include a sensor interface 508, one or more processors 510, a display/graphical user interface ("GUI") 512, a communications interface 514, a memory 516, and a power source (or power connection) 518, all communicating over an internal bus 519. The sensor interface 508 may be implemented in hardware or combination of hardware and software and is used to connect via wired and/or wireless connections to the sensors 504.

The warming therapy device 105 may be attached to one or more of several different types of sensors 504 and may be configured to measure and readout physiological data related to neonatal patient. As noted, the sensors 504 may be attached to the warming therapy device 105 by the conductive leads (not separately shown) which may be, for example, cables coupled to sensor interface 508. Additionally, or alternatively, one or more sensors 504 may be connected to sensor interface 508 via a wireless connection. In which case sensor interface 508 may include circuity for receiving data from and sending data to one or more devices using, for example, a WIFI^{®} connection, a cellular network connection, and/or a BLUETOOTH@ connection. Furthermore, some embodiments may employ separate sensor interface circuits for different types of sensors.

The sensors 504 communicate with the warming therapy device 105 over the communications connections 525, which may be wired, wireless, or a combination thereof. The sensors 504 include one or more environmental condition sensors 545 to sense environmental parameters of the care environment, one or more patient condition sensors 546 to sense patient condition parameters, and one or more warming therapy device condition sensors 547 to sense operational state of the warming therapy device 105.

The environmental condition sensors 545 sense the parameters of the environment that define the environment in which the neonatal patient is kept. These parameters may be set by a caregiver using controls (not shown) on the warming therapy device 105 and, in some embodiments, automatically controlled by the warming therapy device 105. Such automatic control is performed by electronics and programming not shown herein. The environmental parameters typically include quantities such as humidity, oxygen content, and air temperature, among others, and combinations thereof. In the illustrated embodiment, the environmental condition sensors 545 include a humidity sensor 530, an oxygen sensor 531, and an air temperature sensor 532.

The patient condition sensors 546 sense parameters that are monitored and used to define the neonatal patient's condition. These parameters frequently include, by way of non-limiting example, blood oxygen saturation ("SpO2"), non-invasive blood pressure ("NIBP"), end-tidal carbon dioxide ("etCO2"), or body (or, "core") temperature, or skin temperature, or skin galvanic response, or motion, or combinations thereof. The illustrated embodiment also measures galvanic response, which is not monitored or used to define the patient's condition in conventional practice. The patient condition sensors 546 in the illustrated embodiment include a blood oxygen saturation sensor 533, body temperature sensor 534, skin temperature sensor 535, galvanic response 410, motion sensor 536, and blood pressure sensor 537.

The warming therapy device condition sensors 547 sense various potential conditions of the warming therapy device 105 itself that may be of interest. Again, non-limiting examples may include whether there is power to the warming therapy device 105, whether the warming therapy device 105 is turned on, whether the warming therapy device 105 is closed, or the status of the door latch for the warming therapy device 105, , or the settings for the warming therapy device 105, or combinations thereof. Accordingly, the warming therapy device condition sensors 547 in the illustrated embodiment include power sensor 538, a power-on sensor 539, a door closed sensor 540, a latch status sensor 541, and a settings sensor(s) 542.

Still referring to FIG. 5, the sensors 504 sense their respective parameters, generate signals representative thereof, and transmit them over the interconnections 525 to the warming therapy device 105. The processor(s) 510 handle the received data signals under the programmed control of instructions residing on the memory 516. The handling may include displaying the data on the display/GUI 512, analyzing the data signals as discussed further below, and transmitting the data to the central monitoring station 115, shown in FIG. 1.

The one or more processors 510 may be used for controlling the general operations of the warming therapy device 105, as well as processing sensor data received by sensor interface 508. The one or more processors 510 may be any suitable processor-based resource. They may be, but are not limited to, a central processing unit ("CPU"), a hardware microprocessor, a multi-core processor, a single core processor, a field programmable gate array ("FPGA"), a microcontroller, an application specific integrated circuit ("ASIC"), a digital signal processor ("DSP"), or other similar processing device capable of executing any type of instructions, algorithms, or software for controlling the operation and performing the functions of warming therapy device 105. In some embodiments, the one or more processors 510 may comprise a processor chipset including, for example and without limitation, one or more co-processors.

The display/GUI 512 may be configured to display various patient data, sensor data, and hospital or patient care data, and includes a user interface implemented for allowing interaction and communication between a user and warming therapy device 105. The display/GUI 512 may include a keyboard (not shown) and/or pointing or tracking device (not shown), as well as a display, such as a liquid crystal display ("LCD"), cathode ray tube ("CRT") display, thin film transistor ("TFT") display, light-emitting diode ("LED") display, high definition ("HD") display, or other similar display device that may include touch screen capabilities. The display/GUI 512 may provide a means for inputting instructions or information directly to the warming therapy device 105. The patient information displayed may, for example, relate to the measured physiological parameters of the neonatal patient.

The communications interface 514 may permit the warming therapy device 105 to directly or indirectly (via, for example, a monitor mount) communicate with one or more computing networks and devices, workstations, consoles, computers, monitoring equipment, alert systems, and/or mobile devices (e.g., a mobile phone, tablet, or other hand-held display device). The communications interface 514 may include various network cards, interfaces, communication channels, cloud, antennas, and/or circuitry to permit wired and wireless communications with such computing networks and devices. The communications interface 514 may be used to implement, for example, a BLUETOOTH^{®} connection, a cellular network connection, and/or a WIFI^{®} connection with such computing networks and devices. Example wireless communication connections implemented using the communication interface 514 include wireless connections that operate in accordance with, but are not limited to, IEEE802.11 protocol, a Radio Frequency For Consumer Electronics ("RF4CE") protocol, and/or IEEE802.15.4 protocol (e.g., ZigBee^{®} protocol). In essence, any wireless communication protocol may be used.

Additionally, the communications interface 514 may permit direct (i.e., device-to-device) communications (e.g., messaging, signal exchange, etc.) such as from a monitor mount to warming therapy device 105 using, for example, a universal serial bus ("USB") connection or other communication protocol interface. The communication interface 514 may also permit direct device-to-device connection to other devices such as to a tablet, computer, or similar electronic device; or to an external storage device or memory.

The memory 516 may be a single memory device or one or more memory devices at one or more memory locations that may include, without limitation, one or more of a random-access memory ("RAM"), a memory buffer, a hard drive, a database, an erasable programmable read only memory ("EPROM"), an electrically erasable programmable read only memory ("EEPROM"), a read only memory ("ROM"), a flash memory, hard disk, various layers of memory hierarchy, or any other non-transitory computer readable medium. The memory 516 may be on-chip, or off-chip, or a combination of the two, depending on the implementation of the one or more processors 510. The memory 516 may be volatile, or non-volatile, or a combination thereof. Similarly, the memory 516 may be installed, or remote, or a combination thereof. The memory 516 may be used to store any type of instructions and patient data associated with algorithms, processes, or operations for controlling the general functions and operations of the warming therapy device 105.

The power source 518 may include a self-contained power source such as a battery pack and/or include an interface to be powered through an electrical outlet, either directly or by way of a monitor mount. The power source 518 may also be a rechargeable battery that can be detached allowing for replacement. In the case of a rechargeable battery, a small built-in back-up battery (or super capacitor) can be provided for continuous power to be provided to the warming therapy device 105 during battery replacement. Communication between the components of the warming therapy device 105 in this example (may be established using the internal bus 519.

The data signals received from the sensors 504 may be analog signals. Some sensors and/or their leads may include amplifying and filtering circuity as well as analog-to-digital (A/D) circuity that converts the analog signal to a digital signal using amplification, filtering, and A/D conversion methods. In the event that one or more of the sensors 504 is a wireless sensor, the sensor interface 508 may receive the data signals from a wireless communication module (not shown in FIG. 5). Thus, the sensor interface 508 is a component which may be configured to interface with the one or more sensors 504 and receive sensor data therefrom.

The one or more processors 510, along with some or all of the memory 516, may constitute a warming therapy device controller. The warming therapy device controller may analyze the received data signal waveforms to identify certain waveform characteristics and threshold levels indicative of conditions (abnormal and normal) of the neonatal patient using one or more monitoring methods as described herein. A monitoring method may include comparing an analog or a digital waveform characteristic or an analog or digital value to one or more threshold values and generating a comparison.

Returning now to FIG. 1, the warming therapy devices 105 are deployed within the context of a larger patient monitoring system 100. The computing system 120 may be a distributed computing environment constituting and/or including a networked, cloud, or peer-to-peer structure. In the illustrated embodiment, the computing system 120 is a network over which the warming therapy devices 105 push and/or pull data and information. The communications among the warming therapy device 105, the computing system 306, and other resources of the patient monitoring system 100 may be either wireless or wired depending upon the technical capabilities of the various components.

For example, the medical facility 101 in which the neonatal patient is located may include a central monitoring station 309 from which a caregiver 125 may monitor the physical condition of multiple neonatal patients concurrently. The warming therapy device 105, using, for example, the communications interface 514 shown in FIG. 5, may push sensed physiological parameters and/or signals representative thereof to the central monitoring station 115 over the computing system 306. The pushed data and information may then be displayed for the caregiver 125 for review and consideration.

For another example, the warming therapy devices 105 may pull information from one or more electronic medical record ("ERM") 130 for the neonatal patient from a records repository 135 using, for example, the communications interface 114 shown in FIG. 1. The pulled information can then be displayed by the warming therapy device 105. Such pulled information might include, depending on the implementation and without limitation, medication regimens, diagnosed medical conditions, medical history, historical medical data, etc. Similarly, data sensed by or entered at the warming therapy device 105 may be pushed to, for instance, the ERM 130 for storage and later retrieval.

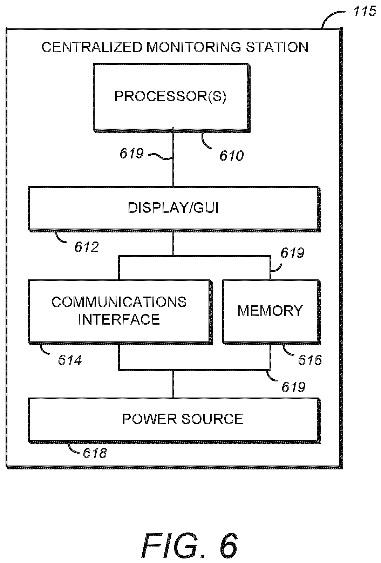

FIG. 6 schematically illustrates a central monitoring station first shown in FIG. 1. Again, in general, it is contemplated by the present disclosure that centralized monitoring station 115 includes electronic components and/or electronic computing devices operable to receive, transmit, process, store, and/or manage patient data and information associated performing the functions of the system as described herein, which encompasses any suitable processing device adapted to perform computing tasks consistent with the execution of computer-readable instructions stored in a memory or a computer-readable recording medium.

Further, any, all, or some of the computing devices in centralized monitoring station 115 may be adapted to execute any operating system, including Linux^{®}, UNIX^{®}, Windows Server^{®}, *etc.,* as well as virtual machines adapted to virtualize execution of a particular operating system, including customized and proprietary operating systems. Centralized monitoring station 115 may be further equipped with components to facilitate communication with other computing devices over one or more network connections, which may include connections to local and wide area networks, wireless and wired networks, public and private networks, and any other communication network enabling communication in the system.

FIG. 6 schematically illustrates the electronics organization of one particular implementation of the centralized monitoring station 115. As shown in FIG. 6, centralized monitoring station 115 may include one or more processors 610, a display/graphical user interface ("GUI") 612, a communications interface 614, a memory 616, and a power source (or power connection) 618, all communicating over an internal bus 619.

The one or more processors 610 may be used for controlling the general operations of the centralized monitoring station 115, as well as processing sensor data received from the warming therapy devices 105 over the computing system 120. The one or more processors 610 may be any suitable processor-based resource. They may be, but are not limited to, a central processing unit ("CPU"), a hardware microprocessor, a multi-core processor, a single core processor, a field programmable gate array ("FPGA"), a microcontroller, an application specific integrated circuit ("ASIC"), a digital signal processor ("DSP"), or other similar processing device capable of executing any type of instructions, algorithms, or software for controlling the operation and performing the functions of centralized monitoring station 115. In some embodiments, the one or more processors 610 may comprise a processor chipset including, for example and without limitation, one or more co-processors.

The display/GUI 612 may be configured to display various patient data, sensor data, and hospital or patient care data, and includes a user interface implemented for allowing interaction and communication between a user and centralized monitoring station 115. The display/GUI 612 may include a keyboard (not shown) and/or pointing or tracking device (not shown), as well as a display, such as a liquid crystal display ("LCD"), cathode ray tube ("CRT") display, thin film transistor ("TFT") display, light-emitting diode ("LED") display, high definition ("HD") display, or other similar display device that may include touch screen capabilities. The display/GUI 612 may provide a means for inputting instructions or information directly to the centralized monitoring station 115. The patient information displayed may, for example, relate to the measured physiological parameters of the neonatal patient.

The communications interface 614 may permit the centralized monitoring station 115 to directly or indirectly (via, for example, a monitor mount) communicate with one or more computing networks and devices, workstations, consoles, computers, monitoring equipment, alert systems, and/or mobile devices (e.g., a mobile phone, tablet, or other hand-held display device). The communications interface 614 may include various network cards, interfaces, communication channels, cloud, antennas, and/or circuitry to permit wired and wireless communications with such computing networks and devices. The communications interface 614 may be used to implement, for example, a BLUETOOTH^{®} connection, a cellular network connection, and/or a WIFI^{®} connection with such computing networks and devices. Example wireless communication connections implemented using the communication interface 614 include wireless connections that operate in accordance with, but are not limited to, IEEE802.11 protocol, a Radio Frequency For Consumer Electronics ("RF4CE") protocol, and/or IEEE802.15.4 protocol (e.g., ZigBee^{®} protocol). In essence, any wireless communication protocol may be used.

Additionally, the communications interface 614 may permit direct (i.e., device-to-device) communications (e.g., messaging, signal exchange, etc.) such as from a monitor mount to centralized monitoring station 115 using, for example, a universal serial bus ("USB") connection or other communication protocol interface. The communication interface 614 may also permit direct device-to-device connection to other devices such as to a tablet, computer, or similar electronic device; or to an external storage device or memory.

The memory 616 may be a single memory device or one or more memory devices at one or more memory locations that may include, without limitation, one or more of a random-access memory ("RAM"), a memory buffer, a hard drive, a database, an erasable programmable read only memory ("EPROM"), an electrically erasable programmable read only memory ("EEPROM"), a read only memory ("ROM"), a flash memory, hard disk, various layers of memory hierarchy, or any other non-transitory computer readable medium. The memory 616 may be on-chip, or off-chip, or a combination of the two, depending on the implementation of the one or more processors 610. The memory 616 may be volatile, or non-volatile, or a combination thereof. Similarly, the memory 616 may be installed, or remote, or a combination thereof. The memory 616 may be used to store any type of instructions and patient data associated with algorithms, processes, or operations for controlling the general functions and operations of the centralized monitoring station 115.

The power source 618 may include a self-contained power source such as a battery pack and/or include an interface to be powered through an electrical outlet, either directly or by way of a monitor mount. The power source 618 may also be a rechargeable battery that can be detached allowing for replacement. In the case of a rechargeable battery, a small built-in back-up battery (or super capacitor) can be provided for continuous power to be provided to the centralized monitoring station 115 during battery replacement. Communication between the components of the centralized monitoring station 115 in this example (may be established using the internal bus 619.

The one or more processors 610, along with some or all of the memory 616, may constitute a centralized monitoring station controller. The centralized monitoring station controller may analyze the received data signal waveforms to identify certain waveform characteristics and threshold levels indicative of conditions (abnormal and normal) of the neonatal patient using one or more monitoring methods as described herein. A monitoring method may include comparing an analog or a digital waveform characteristic or an analog or digital value to one or more threshold values and generating a comparison.

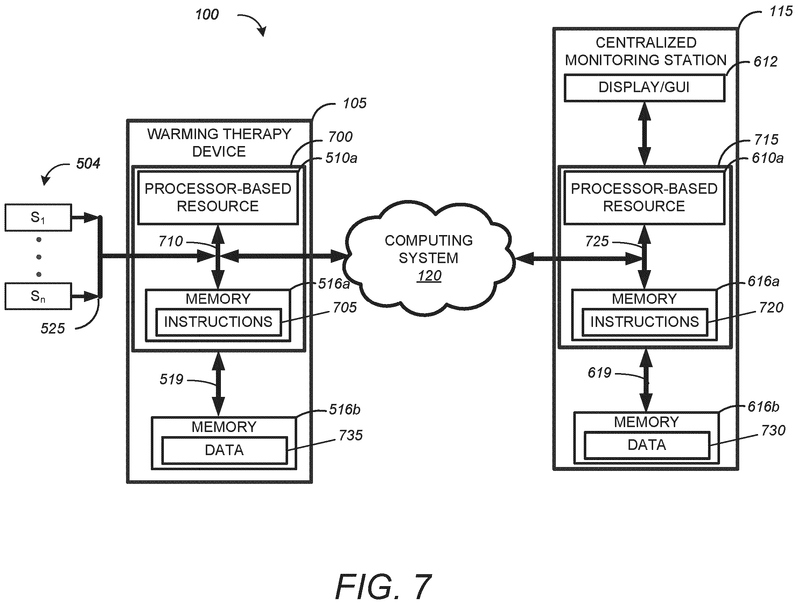

FIG. 7 illustrates one particular embodiment of the patient monitoring system 100 of FIG. 1 highlighting certain aspects thereof to further an understanding of the invention. As described previously, the patient monitoring system 100 includes a plurality of warming therapy devices 105, only one of which is shown, communicating with a centralized monitoring station 115 over a computing system 120. The patient monitoring system 100 also includes a plurality of sensors 504 communicating with the warming therapy devices 105 over their respective communications connections 525.

The communications among the components of the patient monitoring system 100 may be wired or wireless, as mentioned above. Wired communications may implement any suitable communications protocol, such as ETHERNET^{®}. Wireless communications may implement any suitable communications protocol such as WIFI^{®}, BLUETOOTH^{®}, or even some wireless telephony technology, such as a cellular networking protocol. The wireless communication connections can allow, for example, patient and hospital information, alerts, and physiological data to be transmitted in real-time within a hospital wireless communications network (e.g., WIFI^{®}) as well as allow for patient and hospital information, alerts, and physiological data to be transmitted in real-time to other devices.

It is also contemplated by the present disclosure that the communication connections permit communications over other types of wireless networks using alternate hospital wireless communications such as wireless medical telemetry service ("WMTS"), which can operate at specified frequencies (e.g., 1.4 GHz). Other wireless communication connections can include wireless connections that operate in accordance with, but are not limited to, IEEE802.11 protocol, a Radio Frequency For Consumer Electronics (RF4CE) protocol, ZigBee protocol, and/or IEEE802.15.4 protocol.

The warming therapy devices 105 exhibit programmed behaviors as described below implemented by the warming therapy device controller 700. The warming therapy device controller 700 includes a processor-based resource 510a, which, in this particular embodiment, is one of the one or more processors 510 first shown in FIG. 5. The processor-based resource 510a is programmed by instructions 705 residing in a portion 516a of the memory 516, first shown in FIG. 5. The processor-based resource 510a and the memory portion 516a communicate over the internal bus 710.

The centralized monitoring station 115 similarly exhibits programmed behaviors as described below implemented by a centralized monitoring station controller 715. The centralized monitoring station controller 715 includes a processor-based resource 610a, which, in this particular embodiment, is one of the one or more processors 610 first shown in FIG. 6. The processor-based resource 510a is programmed by instructions 720 residing in a portion 616a of the memory 516, first shown in FIG. 5. The processor-based resource 610a and the memory portion 616a communicate over the internal bus 725.

Accordingly, the processor-based resources 510a, 610a perform certain tasks as described herein by executing the respective instructions 705, 720. This may include executing one or more portions of the method described below. However, depending on the environment, the processor-based resources 510a, 610a may also perform other tasks such as pushing data to other computing resources, transmitting alarms, etc. Thus, in this sense, the processor-based resources 510a, 610a, as well as warming therapy devices 105 and/or centralized monitoring station 115 as a whole, may be considered to be "programmed" or "configured" to perform certain functions as described herein.

In the description that follows, the disclosed programmed behaviors of the warming therapy device controllers 700 and the centralized monitoring station controller 715 are performed in real-time, or at least near real-time. The term "near real-time" as used herein means as close to real-time as the computing resources of a given embodiment permit. Accordingly, in some embodiments, the warming therapy device 105 and the centralized monitoring station 115 may include second memory portions 516b, 616b, respectively, in which data 730, 735 and other information may be temporarily stored, or buffered, to accommodate near real-time performance. The second memory portions 516b, 616b may also be used for longer term, or even archival, storage in some embodiments.

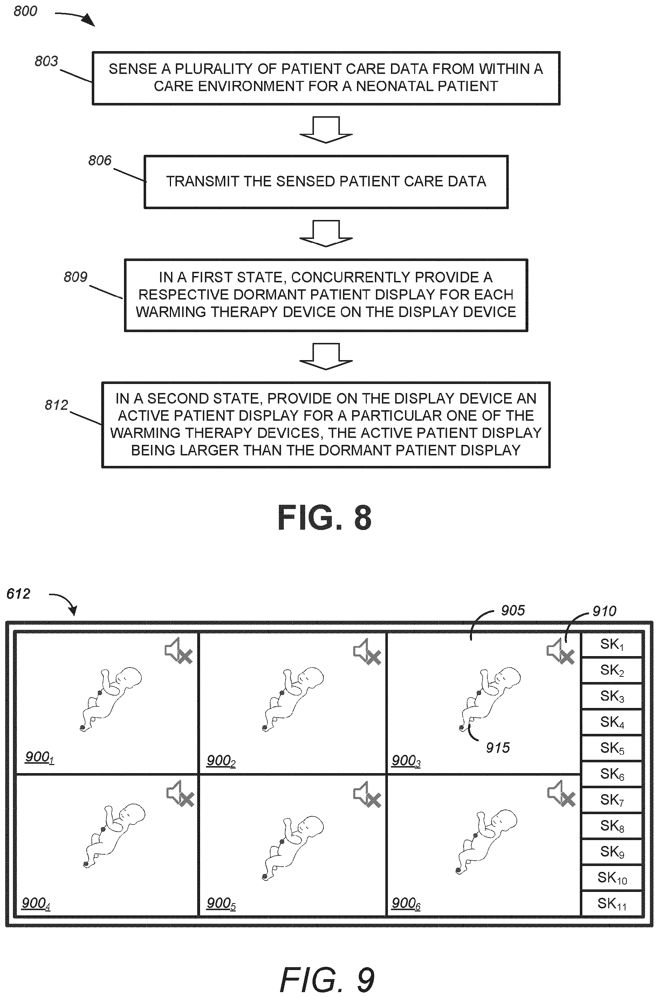

FIG. 8 illustrates a method 800 for neonatal monitoring in accordance with one or more embodiments. The method 800 will be disclosed within the context of the patient monitoring system 100 as shown in FIG. 7. Note, however, that the method is not so limited unless stated to the contrary.

Referring now collectively to FIG. 7 and FIG. 8, the method 800 begins by sensing (at 803) a plurality of patient care data. Patient care data captured in one particular embodiment are listed in Tables 1-3. The sensing is performed by the sensors 504, which may include a variety of different kinds of sensors as discussed above relative to FIG. 5. In the embodiment of FIG. 8, the sensors 504 include a camera and microphone disposed within the care environment defined by the warming therapy device 105 as discussed relative to FIG. 2-FIG. 3 and FIG. 4A -FIG. 4B. The sensors 504 in this particular embodiment also include a galvanic skin response sensor to capture the galvanic electrodermal response of the neonatal patient. Some embodiments, however, may omit sensing the galvanic electrodermal response.

**Table 1. Patient Condition Data**

| Name | Priority | Description |
|---|---|---|
| Body (Core) Temperature | 1 | Also known as central skin temperature, may be supplied by warming therapy device or independent thermometer. |
| Peripheral Skin Temperature | 1 | Supplied by warming therapy device or independent thermometer. |
| Patient Identification | 1 | Information identifying the neonatal patient, such as patient name, patient identification number, location of the warming therapy device. |
| Patient Video Feed | 1 | Visible observation of the neonatal patient. |
| Patient Audio Feed | 1 | Audio observation of the neonatal patient. |
| SpO₂ Telemetry | 2 | Patient blood oxygen saturation. |
| Patient Weight | 2 | Neonatal patient's weight. |
| EEG (or other) Telemetry | 3 | Patient electroencephalogram ("EEG") telemetry. |
| Activity | 4 | Galvanic electrodermal response. |
| Motion | 4 | Neonatal patient motion. |

**Table 2. Environmental Conditions**

| Name | Priority | Description |
|---|---|---|
| Air Temperature | 1 | Air mode temperature setpoint. |
| Skin Temperature | 1 | Skin temperature mode setpoint. |
| Humidity | 1 | Humidity setpoint. |
| Oxygen | 1 | Oxygen level setpoint. |
| SpO2 | 2 | Settings for blood oxygen saturation functional parameters. |
| EEG (or other) Telemetry | 3 | Settings for EEG telemetry. |
| Activity Sensor | 4 | Thresholds for galvanic electrodermal response. |
| Ventilator | 5 | Settings for ventilator operation. |

**Table 3. Warming Therapy Device Data**

| Name | Priority | Description |
|---|---|---|
| Hatch Open | 1 | Hatch to warming therapy device is open. |
| Latch Closed | 1 | Latch for the hatch is closed. |
| Side Panels Open | 1 | Side panels for the warming therapy device are open. |

In Tables 1-3, each entry includes a "priority". In the illustrated embodiments, the priority plays no role in the operation or implementation of the embodiment. However, in some embodiments, the priority may be used to decide priority of hearing/viewing audio and video feeds for the patients located in the warming therapy devices. Priority levels could be set for specific patients in some embodiments based on certain known medical conditions. In some embodiments, priority levels could also be modified by a user as needed based on patient population and known disease states. Some embodiments may implement assorted permutations of these scenarios.

The sensors 504 transmit the sensed data to the warming therapy device controller 700. The warming therapy device controller 700 is programmed to transmit (at 806) the captured patient care data to the centralized monitoring station 115 over the computing system 120. In the illustrated embodiment, the warming therapy device controller 700 is furthermore programmed to analyze the captured patient care data for alarm conditions and if an alarm condition is detected, transmit an alarm signal. The subject matter claimed below admits wide latitude in how the analysis may be performed. It may be as simple as a comparison of sensed values to predetermined threshold values or to more complex analyses.

The transmitted patient care data and, in this embodiment, the transmitted alarm signals (if any), are then received by the centralized monitoring station 115. Ordinarily, the centralized monitoring station controller 715 operates in a first state in which it concurrently provides (at 809) a respective dormant patient display for each warming therapy device 105 on the display device 512, such as is shown in FIG. 9. The display shown in FIG. 9 is but one non-limiting embodiment and it is to be understood that such a display may vary in alternative embodiments.

FIG. 9 illustrates one particular embodiment of a display in the first state on the display device 612 of the centralized monitoring station 115, previously shown in FIG. 6-FIG. 7. The display device 612 includes a single screen, but alternative embodiments may employ multiple screens. The size of the screen is not material, but larger is generally preferred over smaller given the potential amount of information that may be displayed. The technology used to fabricate the display device 612 and its screen are not material. However, it is generally preferable that the screen be a touchscreen although some embodiments may employ peripheral interface devices such as a mouse or trackpad in addition to or *in lieu* of touchscreen technology.

In FIG. 9, the example dormant patient views 900₁-900₆ are shown for six corresponding warming therapy devices (not shown). Using the dormant patient view 900₃ as an example, each dormant patient views 900₁-900₆ includes at least the video imagery 905 from the respective warming therapy device and a button 910. The button 910 may be used by a caregiver to selectively hear captured vocalizations of the neonatal patient 915. In the illustration of FIG. 9, all the buttons 910 show that the audio feed is muted. Also presented are a plurality of soft key controls SK₁-SK₁₁.

More technically, the information presented on the display device 612 is all presented through the graphical user interface ("GUI") previously discussed. Note that this is an implementation-specific detail, and other display techniques may be used. In the context of the GUI, the dormant patient views 900₁-900₆ and soft key controls SK₁-SK₁₁ may be presented as separate windows within the GUI or may be presented as separate panes within a single window, or some combination thereof. For example, each of the dormant patient views 900₁-900₆ may be presented as a plurality of panes within a single window while the soft key controls SK₁-SK₁₁ may be presented as separate buttons within a second, single pane.

The soft key controls SK₁-SK₁₁, where implemented, may perform any number of functions. For example, the soft key controls may be used to control the display, such as by selecting a particular dormant patient view to enlarge for better viewing, or to call up device settings, or to manually trigger an alarm from active observation, or to display information regard system setup or configuration, etc. These kinds of details will all be implementation-specific, as will the number of soft key controls.

Each of the dormant patient views 900₁-900₆ includes video imagery 905 of a neonatal patient 915. This is not required, however. Some embodiments may still present or provide a dormant patient view for a warming therapy device that is empty, or from which the neonatal patient 915 has been temporarily removed. However, by removing the dormant patient view in which no neonatal patient appears will provide additional space to present larger dormant patient views in which the neonatal patient is present. This capability may be implemented in software by the centralized monitoring station controller 715, shown in FIG. 7, or may be selectively exercised by a caregiver through the GUI.

Accordingly, the actual number of dormant patient views at any given time will be implementation specific. The view in FIG. 9 includes six dormant patient views, which may be obtained from six or more warming therapy devices. In alternative embodiments, other numbers of dormant patient views may be presented, and even different numbers at different times while the centralized monitoring station 115 is operating.

Returning now to FIG. 7 and FIG. 8, the central monitoring station 115 operates (at 809) in the first state until something triggers it to operate (at 812) in a second state, wherein the central monitoring station 115 provides on the display device 612 an active patient display for a particular one of the warming therapy devices. The active patient display is larger than the dormant patient display relative to the size of the screen to provide the caregiver a better opportunity for viewing the neonatal patient and whether the neonatal patient is in some kind of distress.

An absolute quantification of the term "larger" within the context of the technique disclosed herein will depend on a number of factors and, therefore, is an implementation specific detail. One limitation on the size of a "larger" active patient display is the size of the screen for the display device 612. Other factors include the size and numbers of the dormant patient displays. For example, on a given screen of constant size, two dormant patient displays may be displayed in a "larger" size-perhaps, roughly 45%-50% of the screen each-than can eight dormant patient views-perhaps, roughly 10%-12.5% of the screen apiece. Thus, when the active patient display is provided, how much "larger" it is than the dormant patient display may be a function of how many dormant patient displays (and their size) were originally displayed.

Thus, how "large" is "larger" will depend on a number of factors characterizing a specific implementation. The larger size of the active patient display relative to the dormant patient display is limited only by the screen of the display device 612, although the active patient display need not necessarily occupy the entirety of the screen. In some embodiments, the active patient display may be only 10% larger than the dormant patient display, whereas the active patient display may be 15% larger in other embodiments, up to perhaps 100% of the dormant patient display. Those skilled in the art having the benefit of this disclosure will appreciate the factors mentioned above, and perhaps others as well, in the implementation of any given embodiment.

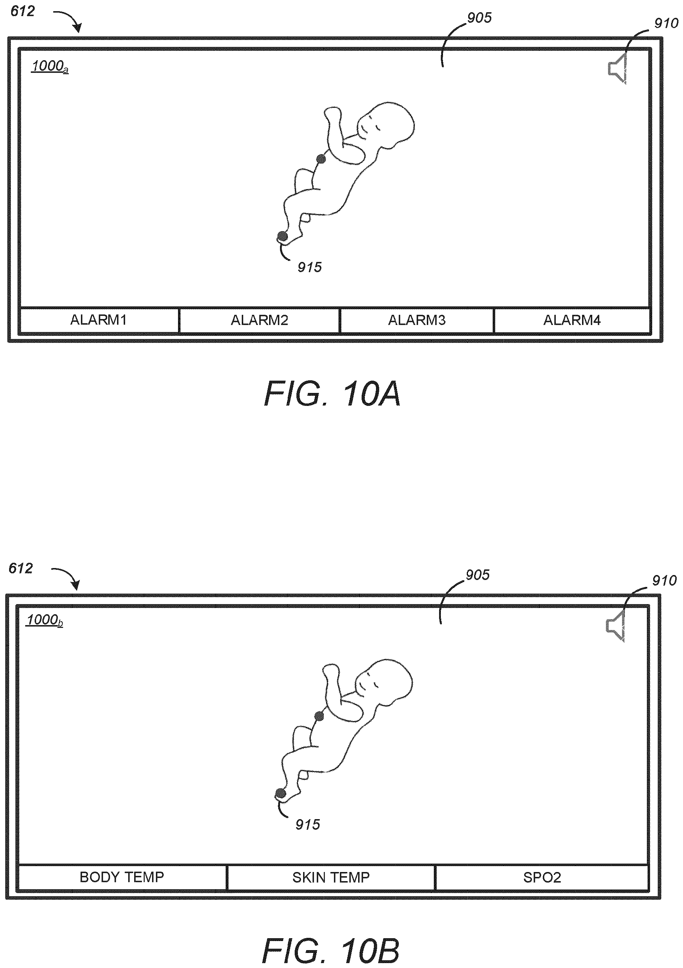

The subject matter claimed below admits wide variation in the composition of the active patient display and the presentation of data/information therein. FIG. 10A-FIG. 10D depict embodiments of the display in a second state of operation for the central monitoring station 115. Note that each of the active patient displays 1000ₐ-1000_{d} includes video imagery 905 of the neonatal patient 915 and an audio button 910, which has been selectively activated by a caregiver.

Note that in each of FIG. 10A-FIG. 10D the respective active patient display 1000ₐ-1000_{d} occupies the entire screen of the display device 612. The intention is to provide a caregiver at the centralized monitoring station 115 a better ability to remotely monitor the condition of the neonatal patient 915. In general, a larger display facilitates this monitoring and, in particular, the visual observation aspect. However, it is not necessary that the active patient display occupy the entire screen of the display device 612, only that it be larger than the respective dormant patient display.

FIG. 10A depicts an active patient display 1000ₐ depicting not only the video imagery 905 and the audio button 910, but also the status of various alarms ALARM1-ALARM4 in soft key controls along the bottom of the display 1000ₐ. The display 1000ₐ may be useful where, for example, a caregiver wishes to obtain a closer visual inspection and selectively triggers the centralized monitoring station controller 715, shown in FIG. 7, to provide the active patient display 1000ₐ. Typically, the soft key controls will bear a descriptive label for the actual alarm. The soft key controls might also indicate the status of the alarm, for example through color coding. In some embodiments, selecting a particular soft key control may display or prompt display of captured patent care data associated with the particular alarm.

FIG. 10B depicts an active patient display 1000_{b} such as may be automatically displayed upon detection of an alarm condition. In some embodiments, the warming therapy device controller 700, shown in FIG. 7, is programmed to analyze captured patient care data to see if any alarm conditions exist and, if so, transmit an alarm. The centralized monitoring station controller 715, upon receiving the transmitted alarm, then automatically provides an active patient display such as the active patient display 1000_{b}. The alarms for one particular embodiment are listed in Table 4. In this context, the term "automatically" means under programmed control and without direct human intervention.

**Table 4. Example Alarms**

| Name | Priority | Description |
|---|---|---|
| Patient Temperature | 1 | Temperature out of range or disconnected, e.g., HI TEMP, LO TEMP, and/or TEMP CUT OUT. |
| Humidity | 1 | Humidity out of range. |
| Oxygen | 1 | Oxygen levels out of range. |
| Hatch | 1 | Hatch unexpectedly open/closed. |
| Side Panels | 1 | Side panels unexpectedly open/closed. |
| Air Temperature | 1 | Air temperature out of range. |
| SpO₂ | 2 | All blood oxygen saturation alarms. |
| EEG (or other) Telemetry | 3 | All cardiac alarms. |
| Activity Sensor | 4 | Change in electrodermal response outside of range. |

As with the other active patient displays 1000ₐ and 1000_{c}-1000_{d}, the active patient display 1000_{b} includes the video imagery 905 and the button 910 has been selected to provide the vocalizations of the neonatal patient 915. Also included along the bottom of the display are the body temperature, skin temperature, and blood oxygen saturation ("SPO2"). In this embodiment, the active patient display 1000_{b} is displaying patient care information associated with or relevant to the alarm condition. Thus, in this example, perhaps the neonatal patient 905 may be experiencing a fever, hyperthermia, or hypothermia that resulted in the alarm. Note that the numerical values of the sensed parameters will typically be presented with or without some kind of label, although in some embodiments there may be soft keys controls that may be selected to display the desired information.

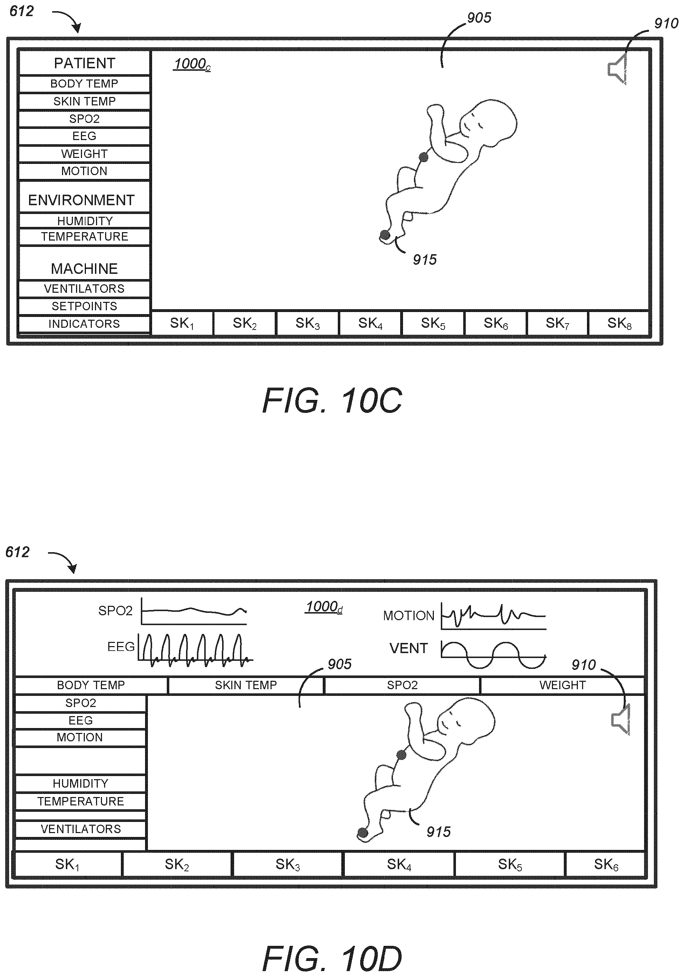

FIG. 10C depicts an active patient display 1000_{c} that might be useful in situations in which the caregiver has selected a particular dormant patient view for closer observation of the neonatal patient 915. In addition to the soft key controls SK₁-SK₈, the active patient display 1000_{c} includes a panel along the left side thereof listing a variety of patient condition parameters ("PATIENT"), environmental condition parameters ("ENVIRONMENT"), and warming therapy device parameters ("MACHINE"). The values themselves may be displayed or the values may be displayed by selection of buttons in the panel.

The information displayed may be in whatever form is most convenient and effective. In FIG. 10A-FIG. 10C, except for the video imagery 905 and the audio vocalizations, the information presented is in alpha-numeric text. In the active patient display 1000_{d} of FIG. 10D, data for SPO2, motion, electroencephalogram ("EEG"), and the ventilators ("VENT"), are shown as graphed waveforms. In some embodiments, some patient care data may be presented graphically, some in alphanumeric text. In other embodiments, it may all be presented in alphanumeric text. In still other embodiments, the patient care data may be presented all in graphical form.

The presentation of the patient care data in a graphical form implies the presentation of historical trend data in the active patient display. This presentation of historical trend data may be seen in some embodiments, both graphically and in alphanumeric text. The historical trend data may be stored, for example, as data 730, 735 in the memory portions 516b, 616b, all of which are shown in FIG. 7. As noted earlier, patient care data may be pushed to, for example the ERM 130, shown in FIG. 1, so that historical trend data may later then be pulled therefrom.

In various embodiments, then, the active patient display for any corresponding dormant patient display may be triggered in any one of three ways. First, the active patient display may be triggered and displayed automatically by the receipt of an alarm signal. Second, the active patient display may be triggered by caregiver selection. Third, the active patient display may be triggered by sensor active data (e.g., galvanic skin resistance, body temperature, skin temperature, blood oxygen saturation).

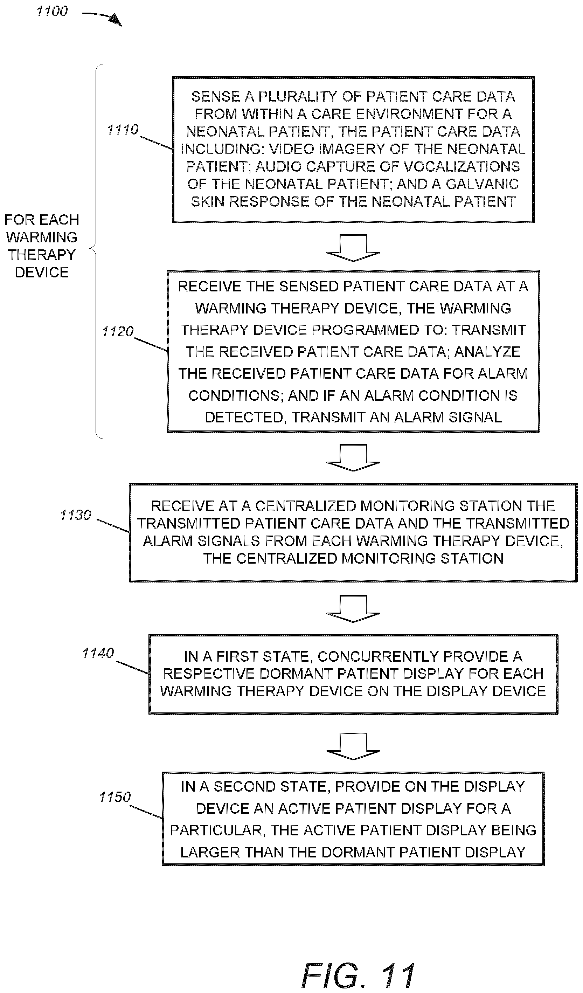

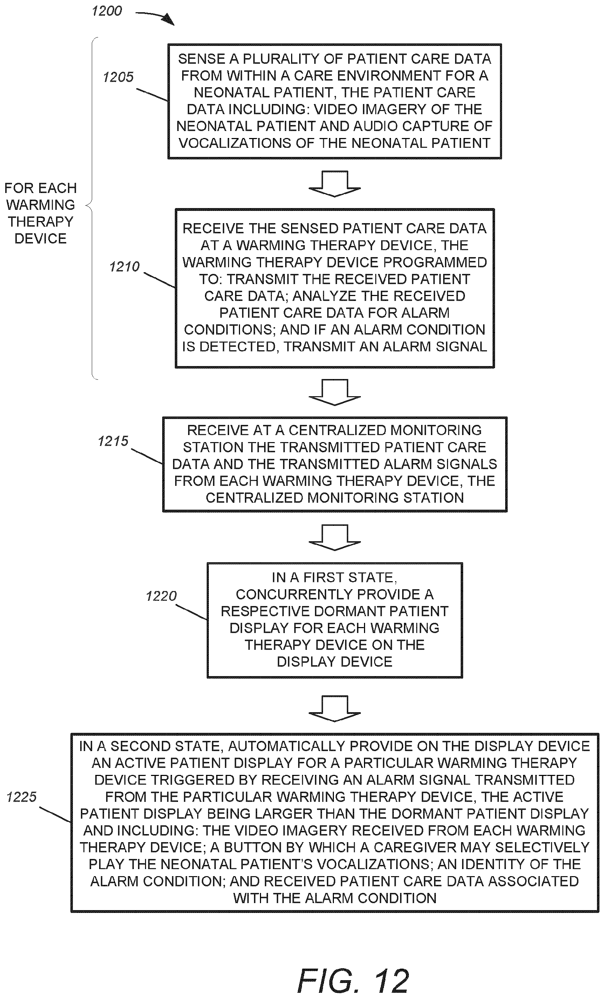

To further an understanding of the presently disclosed technique, two particular embodiments will now by specifically discussed relative to FIG. 11 and FIG. 12. In the embodiment of FIG. 11, the active patient display is selected by a caregiver. In the embodiment of FIG. 12, the warming therapy device 105 detects an alarm condition and transmits an alarm signal that causes the display device 612 of the centralized monitoring station 115 to automatically display the active patient display.

Note that most alarms are based on set limits and would be based on the active measurements within those limits. For example, the skin temperature of the patient is 37°C (98.6°F) for an adult. So, if the infant's measured skin temperature falls to 35°C (95°F), the Low Temperature alarm ("TEMP LO") would activate. Other alarm indicators, for example, Hatch is OPEN would be an alarm type. Air Temperature of the incubator would be based on the Set Point of the closed care environment. For instance, the closed care device is set for 37.5°C (99.5°F). If there were a problem with the air temperature measuring something outside of the set point, the alarm would activate.

Referring now to FIG. 11, a method 1100 for neonatal patient monitoring is illustrated. The method 1100 begins by, for each of a plurality of warming therapy devices, sensing (at 1110) a plurality of patient care data from within a care environment for a neonatal patient. The patient care data in this particular embodiment includes: video imagery of the neonatal patient; audio capture of vocalizations of the neonatal patient; and a galvanic skin response of the neonatal patient. The method 1100 then continues by, for each warming therapy device, receiving (at 1120) the sensed patient care data at a warming therapy device. The warming therapy device in this particular embodiment is programmed to: transmit the received patient care data; analyze the received patient care data for alarm conditions; and if an alarm condition is detected, transmit an alarm signal.

The transmitted patient care data and the transmitted alarm signals from each warming therapy device is then received (at 1130) at a centralized monitoring station. The central monitoring station is programmed to, in a first state, concurrently provide (at 1140) a respective dormant patient display for each warming therapy device on the display device. The central monitoring station is further programmed to, in a second state, provide (at 1150) on the display device an active patient display for a particular, the active patient display being larger than the dormant patient display.

Turning now to FIG. 12, a method 1200 for neonatal monitoring is disclosed. The method 1200 begins by, for each of a plurality of warming therapy devices, sensing (at 1205) a plurality of patient care data from within a care environment for a neonatal patient, the patient care data. The patient care data in this particular embodiment includes video imagery of the neonatal patient and audio capture of vocalizations of the neonatal patient. The method 1200 then, for each warming therapy device, receives (at 1210) the sensed patient care data at a warming therapy device. The warming therapy device is programmed to: transmit the received patient care data; analyze the received patient care data for alarm conditions; and if an alarm condition is detected, transmit an alarm signal.

The method 1200 then receives (at 1215) at a centralized monitoring station the transmitted patient care data and the transmitted alarm signals from each warming therapy device. The centralized transmitting station is programmed to, in a first state, concurrently provide (at 1220) a respective dormant patient display for each warming therapy device on the display device. The centralized monitoring station is further programmed to, in a second state, automatically provide (at 1225) on the display device an active patient display for a particular warming therapy device triggered by receiving an alarm signal transmitted from the particular warming therapy device, the active patient display being larger than the dormant patient display. The active patient display, in this particular embodiment, including: the video imagery received from each warming therapy device; and a button by which a caregiver may selectively play the neonatal patient's vocalizations; an identity of the alarm condition; and received patient care data associated with the alarm condition.

Accordingly, in a first embodiment, a neonatal patient monitoring system comprises a central monitoring system including a display device and a central monitoring controller. The central monitoring controller is programmed to: receive a plurality of transmitted patient care data from each of a plurality of warming therapy devices; in a first state, concurrently provide a respective dormant patient display for each warming therapy device on the display device; and in a second state, provide on the display device an active patient display for a particular one of the warming therapy devices, the active patient display being larger than the dormant patient display.

In a second embodiment, in the neonatal patient monitoring system of the first embodiment, the active patient display is automatically provided upon receipt of an alarm signal from the particular one of the warming therapy devices.

In a third embodiment, in the neonatal patient monitoring system of the second embodiment, the active patient display includes: video imagery received from the particular warming therapy device; a button by which a caregiver may selectively play the neonatal patient's vocalizations; an identity of the alarm condition; and received patient care data associated with the alarm condition.

In a fourth embodiment, in the neonatal patient monitoring system of the first embodiment, the active patient display is provided upon selection of a caregiver.

In a fifth embodiment, in the neonatal patient monitoring system of the first embodiment, the active patient display includes: video imagery received from the particular warming therapy device; a button by which a caregiver may selectively play the neonatal patient's vocalizations; and received patient care data selected by the caregiver.

In a sixth embodiment, in the neonatal patient monitoring system of the first embodiment, the patient care data includes: video imagery received from the particular warming therapy device; a button by which a caregiver may selectively play the neonatal patient's vocalizations; and a skin galvanic response of the neonatal patient.

In a seventh embodiment, the neonatal patient monitoring system of the first embodiment further comprises the plurality of warming therapy devices. Each warming therapy device defines a respective care environment and includes a plurality of sensors to capture patient care data when the warming therapy device is in use, the patient care data and a warming therapy device controller programmed to transmit the captured patient care data, The plurality of sensors includes a camera and a microphone. The camera is positioned in the respective care environment and directed on a patient location to capture video imagery of the neonatal patient. The microphone is positioned in the care environment to capture vocalizations of the neonatal patient.

In an eighth embodiment, in the neonatal patient monitoring system of the seventh embodiment, the plurality of sensors includes a galvanic skin response sensor to capture the galvanic electrodermal response of the neonatal patient.

In a ninth embodiment, in the neonatal patient monitoring system of the seventh embodiment, the warming therapy device controller is programmed to: analyze the captured patient care data for alarm conditions; and if an alarm condition is detected, transmit an alarm signal.

In a tenth embodiment, a method for neonatal monitoring, comprises: receiving a plurality of transmitted patient care data from each of a plurality of warming therapy devices; in a first state, concurrently providing a respective dormant patient display for each warming therapy device on the display device; and in a second state, providing on the display device an active patient display for a particular one of the warming therapy devices, the active patient display being larger than the dormant patient display.

In an eleventh embodiment, in the method of the tenth embodiment, providing the active patient display includes automatically providing upon receipt of an alarm signal from the particular one of the warming therapy devices.

In a twelfth embodiment, in the he method of the eleventh embodiment, providing the active patient display includes providing: video imagery received from the particular warming therapy device; and a button by which a caregiver may selectively play the neonatal patient's vocalizations; an identity of the alarm condition; and received patient care data associated with the alarm condition.

In a thirteenth embodiment, in the method of the tenth embodiment, providing the active patient display includes providing the active patient display upon selection of a caregiver.

In a fourteenth embodiment, in the method of the thirteenth embodiment, providing the active patient display includes providing: video imagery received from the particular warming therapy device; a button by which a caregiver may selectively play the neonatal patient's vocalizations; and received patient care data selected by the caregiver.

In a fifteenth embodiment, the method of the tenth embodiment further comprises sensing the plurality of patient care data by each of the plurality of warming therapy devices and transmitting the patient care data from the warming therapy devices. Sensing the plurality of patient care data includes: acquiring the video imagery from within the care environment; and capturing the neonatal patient's vocalizations from within the care environment.

In a sixteenth embodiment, in the method of the fifteenth embodiment, sensing the plurality of patient care data further includes sensing the galvanic skin response of the neonatal patient.

In a seventeenth embodiment, the method of the fifteenth embodiment further comprises, by each respective warming therapy device: analyzing the captured patient data for alarm conditions; and if an alarm condition is detected, transmitting an alarm signal.

In an eighteenth embodiment, a neonatal patient monitoring system, comprising a plurality of warming therapy devices and a central monitoring station. Each warming therapy device defines a respective care environment and includes a plurality of sensors and a warming therapy device controller. The plurality of sensors capture patient care data when the warming therapy device is in use. The plurality of sensors include a camera, a microphone, and a galvanic skin response sensor. The camera is positioned in the respective care environment and is directed on a patient location to capture video imagery of the neonatal patient. The microphone is positioned in the care environment to capture vocalizations of the neonatal patient. The galvanic skin response sensor to capture the galvanic electrodermal response of the neonatal patient. The warming therapy device controller programmed to: transmit the captured patient care data; analyze the captured patient care data for alarm conditions; and if an alarm condition is detected, transmit an alarm signal. The central monitoring station includes a display device and a central monitoring controller. The central monitoring controller is programmed to: receive the transmitted patient care data and transmitted alarm signals from each warming therapy device; in a first state, concurrently provide a respective dormant patient display for each warming therapy device on the display device; and in a second state, provide on the display device an active patient display for a particular one of the warming therapy devices, the active patient display being larger than the dormant patient display.

In a nineteenth embodiment, in the neonatal patient monitoring system of the eighteenth embodiment, the central monitoring controller is programmed to, in the second state, automatically provide on the display device the active patient display, the second state being triggered by receiving an alarm signal transmitted from the particular warming therapy device.

In a twentieth embodiment, in the neonatal patient monitoring system of the nineteenth embodiment, the active patient display includes: the video imagery received from the respective warming therapy device; a button by which a caregiver may selectively play the neonatal patient's vocalizations; an identity of the alarm condition; and received patient care data associated with the alarm condition.

In a twenty-first embodiment, in the neonatal patient monitoring system of the eighteenth embodiment, the second state includes providing the active patient display is triggered by caregiver selection.

In a twenty-second embodiment, in the neonatal patient monitoring system of the twenty-first embodiment, the active patient display includes: the video imagery received from the respective warming therapy device; a button by which a caregiver may selectively play the neonatal patient's vocalizations; and at least a subset of the received patient care data.

In a twenty-third embodiment, in the neonatal patient monitoring system of the eighteenth embodiment, the at least one of the warming therapy devices is an incubator.

In a twenty-fourth embodiment, in the neonatal patient monitoring system of the eighteenth embodiment, the camera and the microphone are integrated into a single device.

In a twenty-fifth embodiment, in the neonatal patient monitoring system of the eighteenth embodiment, the camera and the microphone are integrated into the respective warming therapy apparatus.

In a twenty-sixth embodiment, in the neonatal patient monitoring system of the eighteenth embodiment, each warming therapy device includes a plurality of sensors. The plurality of sensors includes: one or more environmental condition sensors to sense environmental parameters of the care environment; one or more patient condition sensors to sense patient condition parameters; and one or more warming therapy device condition sensors to sense operational state of the warming therapy device.

In a twenty-seventh embodiment, in the neonatal patient monitoring system of the twenty-sixth embodiment, the environmental condition sensors sense humidity, oxygen levels, or air temperature, or a combination thereof.

In a twenty-eighth embodiment, in the neonatal patient monitoring system of the twenty-sixth embodiment, the patient condition sensors sense blood oxygen saturation ("SpO2"), non-invasive blood pressure ("NIBP"), end-tidal carbon dioxide ("etCO2"), or body temperature, or skin temperature, or skin galvanic response, or motion, or a combination thereof.

In a twenty-ninth embodiment, in the neonatal patient monitoring system of the twenty-sixth embodiment, the warming therapy device condition sensors sense power to the warming therapy device, warming therapy device on, warming therapy device closed, or combinations thereof.

In a thirtieth embodiment, a method for neonatal patient monitoring comprises: for each of a plurality of warming therapy devices, sensing a plurality of patient care data from within a care environment for a neonatal patient, the patient care data including: video imagery of the neonatal patient; audio capture of vocalizations of the neonatal patient; and a galvanic skin response of the neonatal patient; receiving the sensed patient care data at a warming therapy device, the warming therapy device programmed to: transmit the received patient care data; analyze the received patient care data for alarm conditions; and if an alarm condition is detected, transmit an alarm signal; and receiving at a centralized monitoring station the transmitted patient care data and the transmitted alarm signals from each warming therapy device, the centralized monitoring station: in a first state, concurrently provide a respective dormant patient display for each warming therapy device on the display device; and in a second state, provide on the display device an active patient display for a particular, the active patient display being larger than the dormant patient display.

In a thirty-first embodiment, in the method of the thirtieth embodiment, the central monitoring station in the second state automatically provides on the display device the active patient display, the second state being triggered by receiving an alarm signal transmitted from the particular warming therapy device.

In a thirty-second embodiment, in the method of the thirty-first embodiment, the active patient display includes: the video imagery received from the respective warming therapy device; a button by which a caregiver may selectively play the neonatal patient's vocalizations; an identity of the alarm condition; and received patient care data associated with the alarm condition.

In a thirty-third embodiment, in the method of the thirtieth embodiment, in the second state, providing the active patient display is triggered by caregiver selection.

In a thirty-fourth embodiment, in the method of the thirty-third embodiment, the active patient display includes: the video imagery received from the respective warming therapy device; a button by which a caregiver may selectively play the neonatal patient's vocalizations; and at least a subset of the received patient care data.

In a thirty-fifth embodiment, in the method of the thirtieth embodiment, the patient care data includes: one or more environmental parameters of the care environment; one or more patient condition parameters; and one or more operational states of the warming therapy device.

In a thirty-sixth embodiment, in the method of the thirty-fifth embodiment, the environmental parameters include humidity, oxygen levels, or air temperature, or a combination thereof.

In a thirty-seventh embodiment, in the method of the thirty-fifth embodiment, the patient condition parameters include blood oxygen saturation ("SpO2"), non-invasive blood pressure ("NIBP"), end-tidal carbon dioxide ("etCO2"), or body temperature, or skin temperature, or skin galvanic response, or motion, or a combination thereof.

In a thirty-eighth embodiment, in the method of the thirty-fifth embodiment, the warming therapy device condition parameters include power to the warming therapy device, warming therapy device on, warming therapy device closed, or combinations thereof.

In a thirty-ninth embodiment, a neonatal patient monitoring system comprises a plurality of warming therapy devices and a central monitoring station. Each warming therapy device defines a respective care environment and includes a plurality of sensors and a warming therapy device controller. The plurality of sensors capture patient care data when the warming therapy device is in use, the patient care data including video imagery of a neonatal patient and audio data of the neonatal patient's vocalizations. The warming therapy device controller programmed to: transmit the captured patient care data; analyze the captured patient care data for alarm conditions; and if an alarm condition is detected, transmit an alarm signal. The central monitoring station includes a display device and a central monitoring controller. The central monitoring controller is programmed to: receive the transmitted patient care data and transmitted alarm signals from each warming therapy device; in a first state, concurrently provide a respective dormant patient display for each warming therapy device on the display device; and in a second state, automatically provide on the display device an active patient display for a particular warming therapy device triggered by receiving an alarm signal transmitted from the particular warming therapy device, the active patient display being larger than the dormant patient display. The active patient display includes: the video imagery received from each warming therapy device; a button by which a caregiver may selectively play the neonatal patient's vocalizations; an identity of the alarm condition; and received patient care data associated with the alarm condition.

In a fortieth embodiment, in the neonatal patient monitoring system of the thirty-ninth embodiment, each respective dormant patient display includes: the video imagery received from each warming therapy device; and the button by which a caregiver may selectively play the neonatal patient's vocalizations.

In a forty-first embodiment, in the neonatal patient monitoring system of the thirty-ninth embodiment, at least one of the warming therapy devices is an incubator.

In a forty-second embodiment, in the neonatal patient monitoring system of the thirty-ninth embodiment, the plurality of sensors for each warming therapy device includes: a camera by which the video imagery is captured, the camera positioned in the care environment and directed at the neonatal patient location; and a microphone positioned in the care environment by which the neonatal patient's vocalizations are captured.

In a forty-third embodiment, in the neonatal patient monitoring system of the forty-second embodiment, wherein the camera and the microphone are integrated into a single device.

In a forty-fourth embodiment, in the neonatal patient monitoring system of the forty-second embodiment, wherein the camera and the microphone are integrated into the respective warming therapy apparatus.

In a forty-fifth embodiment, in the neonatal patient monitoring system of the thirty-ninth embodiment, the plurality of sensors includes: one or more environmental condition sensors to sense environmental parameters of the care environment; one or more patient condition sensors to sense patient condition parameters; and one or more warming therapy device condition sensors to sense operational state of the warming therapy device.

In a forty-sixth embodiment, in the neonatal patient monitoring system of the forty-fifth embodiment, the environmental condition sensors sense humidity, or oxygen levels, or air temperature, or a combination thereof.

In a forty-seventh embodiment, in the neonatal patient monitoring system of the forty-fifth embodiment, the patient condition sensors sense blood oxygen saturation, or body temperature, or skin temperature, or skin galvanic response, or patient motion, or blood pressure, or a combination thereof.

In a forty-eighth embodiment, in the neonatal patient monitoring system of the forty-fifth embodiment, the warming therapy device condition sensors sense power to the warming therapy device, warming therapy device on, warming therapy device closed, or door latch status, or warming therapy device setting, or combinations thereof.

In a forty-ninth embodiment, in the neonatal patient monitoring system of the thirty-ninth embodiment, the central monitoring controller is further programmed to, in the first state, provide a plurality of soft key controls by which a caregiver may selectively control information presented on the display device.

In a fiftieth embodiment, in the neonatal patient monitoring system of the thirty-ninth embodiment, the central monitoring controller is further programmed to, in the second state, provide a plurality of soft key controls in the active patient display by which a caregiver may selectively control information presented on the display device.

In a fifty-first embodiment, in the neonatal patient monitoring system of the thirty-ninth embodiment, wherein the central monitoring controller is further programmed to, in the first state, provide selected received patient care data in each respective dormant patient display.

In a fifty-second embodiment, in the neonatal patient monitoring system of the thirty-ninth embodiment, the central monitoring controller is further programmed to, in a third state, selectively provide the active patient display on the display device upon selection of the particular warming therapy device by the caregiver.

In a fifty-third embodiment, a method for neonatal monitoring, comprises: for each of a plurality of warming therapy devices, sensing a plurality of patient care data from within a care environment for a neonatal patient, the patient care data including: video imagery of the neonatal patient; and audio capture of vocalizations of the neonatal patient; and receiving the sensed patient care data at a warming therapy device, the warming therapy device programmed to: transmit the received patient care data; analyze the received patient care data for alarm conditions; and if an alarm condition is detected, transmit an alarm signal; and receiving at a centralized monitoring station the transmitted patient care data and the transmitted alarm signals from each warming therapy device, the centralized transmitting station: in a first state, concurrently provide a respective dormant patient display for each warming therapy device on the display device; and in a second state, automatically provide on the display device an active patient display for a particular warming therapy device triggered by receiving an alarm signal transmitted from the particular warming therapy device, the active patient display being larger than the dormant patient display and including: the video imagery received from each warming therapy device; a button by which a caregiver may selectively play the neonatal patient's vocalizations; an identity of the alarm condition; and received patient care data associated with the alarm condition.

In a fifty-fourth embodiment, in the method of the fifty-third embodiment, each respective dormant patient display includes: the video imagery received from each warming therapy device; and the button by which a caregiver may selectively play the neonatal patient's vocalizations.

In a fifty-fifth embodiment, in the method of the fifty-third embodiment, the plurality of sensors for each warming therapy device includes: a camera by which the video imagery is captured, the camera positioned in the care environment and directed at the neonatal patient location; and a microphone positioned in the care environment by which the neonatal patient's vocalizations are captured.

In a fifty-sixth embodiment, in the method of the fifty-third embodiment, the plurality of patient care data includes: one or more environmental parameters of the care environment; one or more patient condition parameters; and one or more operational states of the warming therapy device.

In a fifty-seventh embodiment, in the method of the fifty-third embodiment, the environmental parameters include humidity, or oxygen levels, or air temperature, or a combination thereof.

In a fifty-eighth embodiment, in the method of the fifty-third embodiment, the patient condition parameters include blood oxygen saturation, or body temperature, or skin temperature, or skin galvanic response, or patient motion, or blood pressure, or a combination thereof.

In a fifty-ninth embodiment, in the method of the fifty-third embodiment, the warming therapy device condition parameters include power to the warming therapy device, warming therapy device on, warming therapy device closed, or door latch status, or warming therapy device setting, or combinations thereof.

In a sixtieth embodiment, in the method of the fifty-third embodiment the dormant patient display in the first state includes a plurality of soft key controls by which a caregiver may selectively control information presented on the display device.

In a sixty-first embodiment, in the method of the fifty-third embodiment, the active patient display in the second state includes a plurality of soft key controls in the active patient display by which a caregiver may selectively control information presented on the display device.

In a sixty-second embodiment, in the method of the fifty-third embodiment, the dormant patient display in the first state includes selected received patient care data in each respective dormant patient display.

In a sixty-third embodiment, in the method of the fifty-third embodiment, the central monitoring controller is further programmed to, in a third state, selectively provide the active patient display on the display device upon selection of the particular warming therapy device by the caregiver.

As used herein, expressions such as "include" and "may include" which may be used in the present disclosure denote the presence of the disclosed functions, operations, and constituent elements, and do not limit the presence of one or more additional functions, operations, and constituent elements. In the present disclosure, terms such as "include" and/or "have", may be construed to denote a certain characteristic, number, operation, constituent element, component or a combination thereof, but should not be construed to exclude the existence of or a possibility of the addition of one or more other characteristics, numbers, operations, constituent elements, components or combinations thereof.

As used herein, the article "a" is intended to have its ordinary meaning in the patent arts, namely "one or more." Herein, the term "about" when applied to a value generally means within the tolerance range of the equipment used to produce the value, or in some examples, means plus or minus 10%, or plus or minus 5%, or plus or minus 1%, unless otherwise expressly specified. Further, herein the term "substantially" as used herein means a majority, or almost all, or all, or an amount with a range of about 51% to about 100%, for example. Moreover, examples herein are intended to be illustrative only and are presented for discussion purposes and not by way of limitation.

As used herein, to "provide" an item means to have possession of and/or control over the item. This may include, for example, forming (or assembling) some or all of the item from its constituent materials and/or, obtaining possession of and/or control over an already-formed item.

Unless otherwise defined, all terms including technical and/or scientific terms used herein have the same meaning as commonly understood by one of ordinary skill in the art to which the present disclosure pertains. In addition, unless otherwise defined, all terms defined in generally used dictionaries may not be overly interpreted. In the following, details are set forth to provide a more thorough explanation of the embodiments. However, it will be apparent to those skilled in the art that embodiments may be practiced without these specific details. In other instances, well-known structures and devices are shown in block diagram form or in a schematic view rather than in detail in order to avoid obscuring the embodiments. In addition, features of the different embodiments described hereinafter may be combined with each other, unless specifically noted otherwise. For example, variations or modifications described with respect to one of the embodiments may also be applicable to other embodiments unless noted to the contrary.

Further, equivalent or like elements or elements with equivalent or like functionality are denoted in the following description with equivalent or like reference numerals. As the same or functionally equivalent elements are given the same reference numbers in the figures, a repeated description for elements provided with the same reference numbers may be omitted. Hence, descriptions provided for elements having the same or like reference numbers are mutually exchangeable.

It will be understood that when an element is referred to as being "connected" or "coupled" to another element, it can be directly connected or coupled to the other element or intervening elements may be present. In contrast, when an element is referred to as being "directly connected" or "directly coupled" to another element, there are no intervening elements present. Other words used to describe the relationship between elements should be interpreted in a like fashion (e.g., "between" versus "directly between," "adjacent" versus "directly adjacent," etc.).

In the present disclosure, expressions including ordinal numbers, such as "first", "second", and/or the like, may modify various elements. However, such elements are not limited by the above expressions. For example, the above expressions do not limit the sequence and/or importance of the elements. The above expressions are used merely for the purpose of distinguishing an element from the other elements. For example, a first box and a second box indicate different boxes, although both are boxes. For further example, a first element could be termed a second element, and similarly, a second element could also be termed a first element without departing from the scope of the present disclosure.

A sensor refers to a component which converts a physical quantity to be measured to an electric signal, for example, a current signal or a voltage signal. The physical quantity may for example comprise electromagnetic radiation (e.g., photons of infrared or visible light), a magnetic field, an electric field, a pressure, a force, a temperature, a current, or a voltage, but is not limited thereto.

Use of the phrases "capable of," "capable to," "operable to," or "configured to" in one or more embodiments, refers to some apparatus, logic, hardware, and/or element designed in such a way to enable the use of the apparatus, logic, hardware, and/or element in a specified manner. Use of the phrase "exceed" in one or more embodiments, indicates that a measured value could be higher than a pre-determined threshold (e.g., an upper threshold), or lower than a pre-determined threshold (e.g., a lower threshold). When a pre-determined threshold range (defined by an upper threshold and a lower threshold) is used, the use of the phrase "exceed" in one or more embodiments could also indicate a measured value is outside the pre-determined threshold range (e.g., higher than the upper threshold or lower than the lower threshold). The subject matter of the present disclosure is provided as examples of apparatus, systems, methods, circuits, and programs for performing the features described in the present disclosure. However, further features or variations are contemplated in addition to the features described above. It is contemplated that the implementation of the components and functions of the present disclosure can be done with any newly arising technology that may replace any of the above-implemented technologies.

The detailed description is made with reference to the accompanying drawings and is provided to assist in a comprehensive understanding of various example embodiments of the present disclosure. Changes may be made in the function and arrangement of elements discussed without departing from the spirit and scope of the disclosure. Various embodiments may omit, substitute, or add various procedures or components as appropriate. For instance, features described with respect to certain embodiments may be combined in other embodiments. In addition, descriptions of well-known functions and constructions may be omitted for clarity and conciseness. Accordingly, those of ordinary skill in the art will recognize that various changes and modifications of the examples described herein can be made without departing from the spirit and scope of the present disclosure.

Various modifications to the disclosure will therefore be readily apparent to those skilled in the art, and the generic principles defined herein may be applied to other variations without departing from the spirit or scope of the present disclosure. Throughout the present disclosure the terms "example," "examples," or "exemplary" indicate examples or instances and do not imply or require any preference for the noted examples. Thus, the present disclosure is not to be limited to the examples and designs described herein but is to be accorded the widest scope consistent with the principles and novel features disclosed.

The present disclosure includes the subject matter set out in the following numbered Clauses:.
Clause 1. A neonatal patient monitoring system, comprising: central monitoring station, including: a display device, and a central monitoring controller programmed to: receive a plurality of transmitted patient care data from each of a plurality of warming therapy devices; in a first state, concurrently provide a respective dormant patient display for each warming therapy device on the display device; and in a second state, provide on the display device an active patient display for a particular one of the warming therapy devices, the active patient display being larger than the dormant patient display.
Clause 2. The neonatal patient monitoring system of clause 1, wherein the active patient display is automatically provided upon receipt of an alarm signal from the particular one of the warming therapy devices.
Clause 3. The neonatal patient monitoring system of clause 2, wherein the active patient display includes: video imagery received from the particular warming therapy device; a button by which a caregiver may selectively play the neonatal patient's vocalizations; an identity of the alarm condition; and received patient care data associated with the alarm condition.
Clause 4. The neonatal patient monitoring system of clause 1, wherein the active patient display is provided upon selection of a caregiver.
Clause 5. The neonatal patient monitoring system of clause 1, wherein the active patient display includes: video imagery received from the particular warming therapy device; a button by which a caregiver may selectively play the neonatal patient's vocalizations; and received patient care data selected by the caregiver.
Clause 6. The neonatal patient monitoring system of clause 1, wherein the patient care data includes: video imagery received from the particular warming therapy device; and a button by which a caregiver may selectively play the neonatal patient's vocalizations; and a skin galvanic response of the neonatal patient.
Clause 7. The neonatal patient monitoring system of clause 1, further comprising the plurality of warming therapy devices, each warming therapy device defining a respective care environment and including: a plurality of sensors to capture patient care data when the warming therapy device is in use, the patient care data, the plurality of sensors including: a camera positioned in the respective care environment and directed on a patient location to capture video imagery of the neonatal patient; and a microphone positioned in the care environment to capture vocalizations of the neonatal patient; and a warming therapy device controller programmed to transmit the captured patient care data.
Clause 8. The neonatal patient monitoring system of clause 7, wherein the plurality of sensors includes a galvanic skin response sensor to capture the galvanic electrodermal response of the neonatal patient.
Clause 9. The neonatal patient monitoring system of clause 7, wherein the warming therapy device controller is programmed to: analyze the captured patient care data for alarm conditions; and if an alarm condition is detected, transmit an alarm signal.
Clause 10. A method for neonatal monitoring, comprising: receiving a plurality of transmitted patient care data from each of a plurality of warming therapy devices; in a first state, concurrently providing a respective dormant patient display for each warming therapy device on the display device; and in a second state, providing on the display device an active patient display for a particular one of the warming therapy devices, the active patient display being larger than the dormant patient display.
Clause 11. The method of clause 10, wherein providing the active patient display includes automatically providing upon receipt of an alarm signal from the particular one of the warming therapy devices.
Clause 12. The method of clause 11, wherein providing the active patient display includes providing: video imagery received from the particular warming therapy device; a button by which a caregiver may selectively play the neonatal patient's vocalizations; an identity of the alarm condition; and received patient care data associated with the alarm condition.
Clause 13. The method of clause 10, providing the active patient display includes providing the active patient display upon selection of a caregiver.
Clause 14. The method of clause 13, wherein providing the active patient display includes providing: video imagery received from the particular warming therapy device; a button by which a caregiver may selectively play the neonatal patient's vocalizations; and received patient care data selected by the caregiver.
Clause 15. The method of clause 10, further comprising: sensing the plurality of patient care data by each of the plurality of warming therapy devices, including: acquiring the video imagery from within the care environment; and capturing the neonatal patient's vocalizations from within the care environment; and transmitting the patient care data from the warming therapy devices.
Clause 16. The method of clause 15, wherein sensing the plurality of patient care data further includes sensing the galvanic skin response of the neonatal patient.
Clause 17. The method of clause 15, further comprising by each respective warming therapy device: analyzing the captured patient data for alarm conditions; and if an alarm condition is detected, transmitting an alarm signal.
Clause 18. A neonatal patient monitoring system, comprising: a plurality of warming therapy devices, each warming therapy device defining a respective care environment and including: a plurality of sensors to capture patient care data when the warming therapy device is in use, the plurality of sensors including: a camera positioned in the respective care environment and directed on a patient location to capture video imagery of the neonatal patient; a microphone positioned in the care environment to capture vocalizations of the neonatal patient; and a galvanic skin response sensor to capture the galvanic electrodermal response of the neonatal patient; a warming therapy device controller programmed to: transmit the captured patient care data; analyze the captured patient care data for alarm conditions; and if an alarm condition is detected, transmit an alarm signal; and a central monitoring station, including: a display device, and a central monitoring controller programmed to: receive the transmitted patient care data and transmitted alarm signals from each warming therapy device; in a first state, concurrently provide a respective dormant patient display for each warming therapy device on the display device; and in a second state, provide on the display device an active patient display for a particular one of the warming therapy devices, the active patient display being larger than the dormant patient display.
Clause 19. The neonatal patient monitoring system of clause 18, wherein the central monitoring controller is programmed to, in the second state, automatically provide on the display device the active patient display, the second state being triggered by receiving an alarm signal transmitted from the particular warming therapy device.
Clause 20. The neonatal patient monitoring system of clause 19, wherein the active patient display includes: the video imagery received from the respective warming therapy device; a button by which a caregiver may selectively play the neonatal patient's vocalizations; an identity of the alarm condition; and received patient care data associated with the alarm condition.
Clause 21. The neonatal patient monitoring system of clause 18, wherein, in the second state, providing the active patient display is triggered by caregiver selection.
Clause 22. The neonatal patient monitoring system of clause 21, wherein the active patient display includes: the video imagery received from the respective warming therapy device; a button by which a caregiver may selectively play the neonatal patient's vocalizations; and at least a subset of the received patient care data.
Clause 23. The neonatal patient monitoring system of clause 18, wherein the at least one of the warming therapy devices is an incubator.
Clause 24. The neonatal patient monitoring system of clause 18, wherein the camera and the microphone are integrated into a single device.
Clause 25. The neonatal patient monitoring system of clause 18, wherein the camera and the microphone are integrated into the respective warming therapy apparatus.
Clause 26. The neonatal patient monitoring system of clause 18, wherein each warming therapy device includes: a plurality of sensors, including: one or more environmental condition sensors to sense environmental parameters of the care environment; one or more patient condition sensors to sense patient condition parameters; and one or more warming therapy device condition sensors to sense operational state of the warming therapy device.
Clause 27. The neonatal patient monitoring system of clause 26, wherein the environmental condition sensors sense humidity, oxygen levels, or air temperature, or a combination thereof.
Clause 28. The neonatal patient monitoring system of clause 26, wherein the patient condition sensors sense blood oxygen saturation ("SpO2"), non-invasive blood pressure ("NIBP"), end-tidal carbon dioxide ("etCO2"), or body temperature, or skin temperature, or skin galvanic response, or motion, or a combination thereof.
Clause 29. The neonatal patient monitoring system of clause 26, wherein the warming therapy device condition sensors sense power to the warming therapy device, warming therapy device on, warming therapy device closed, or combinations thereof.
Clause 30. A method for neonatal patient monitoring, comprising: for each of a plurality of warming therapy devices, sensing a plurality of patient care data from within a care environment for a neonatal patient, the patient care data including: video imagery of the neonatal patient; audio capture of vocalizations of the neonatal patient; and a galvanic skin response of the neonatal patient; receiving the sensed patient care data at a warming therapy device, the warming therapy device programmed to: transmit the received patient care data; analyze the received patient care data for alarm conditions; and if an alarm condition is detected, transmit an alarm signal; and receiving at a centralized monitoring station the transmitted patient care data and the transmitted alarm signals from each warming therapy device, the centralized monitoring station: in a first state, concurrently provide a respective dormant patient display for each warming therapy device on the display device; and in a second state, provide on the display device an active patient display for a particular, the active patient display being larger than the dormant patient display.
Clause 31. The method of clause 30, wherein, in the second state, the central monitoring station automatically provides on the display device the active patient display, the second state being triggered by receiving an alarm signal transmitted from the particular warming therapy device.
Clause 32. The method of clause 31, wherein the active patient display includes: the video imagery received from the respective warming therapy device; a button by which a caregiver may selectively play the neonatal patient's vocalizations; an identity of the alarm condition; and received patient care data associated with the alarm condition.
Clause 33. The method of clause 30, wherein, in the second state, providing the active patient display is triggered by caregiver selection.
Clause 34. The method of clause 33, wherein the active patient display includes: the video imagery received from the respective warming therapy device; a button by which a caregiver may selectively play the neonatal patient's vocalizations; and at least a subset of the received patient care data.
Clause 35. The method of clause 30, wherein the patient care data includes: one or more environmental parameters of the care environment; one or more patient condition parameters; and one or more operational states of the warming therapy device.
Clause 36. The method of clause 35, wherein the environmental parameters include humidity, oxygen levels, or air temperature, or a combination thereof.
Clause 37. The method of clause 35, wherein the patient condition parameters include blood oxygen saturation ("SpO2"), non-invasive blood pressure ("NIBP"), end-tidal carbon dioxide ("etCO2"), or body temperature, or skin temperature, or skin galvanic response, or motion, or a combination thereof.
Clause 38. The method of clause 35, wherein the warming therapy device condition parameters include power to the warming therapy device, warming therapy device on, warming therapy device closed, or combinations thereof.
Clause 39. A neonatal patient monitoring system, comprising:
   a plurality of warming therapy devices, each warming therapy device defining a respective care environment and including: a plurality of sensors to capture patient care data when the warming therapy device is in use, the patient care data including video imagery of a neonatal patient and audio data of the neonatal patient's vocalizations; and a warming therapy device controller programmed to: transmit the captured patient care data; analyze the captured patient care data for alarm conditions; and if an alarm condition is detected, transmit an alarm signal; and a central monitoring station,
   including: a display device, and a central monitoring controller programmed to: receive the transmitted patient care data and transmitted alarm signals from each warming therapy device; in a first state, concurrently provide a respective dormant patient display for each warming therapy device on the display device; and in a second state,
   automatically provide on the display device an active patient display for a particular warming therapy device triggered by receiving an alarm signal transmitted from the particular warming therapy device, the active patient display being larger than the dormant patient display and including: the video imagery received from each warming therapy device; a button by which a caregiver may selectively play the neonatal patient's vocalizations; an identity of the alarm condition; and received patient care data associated with the alarm condition.
Clause 40. The neonatal patient monitoring system of clause 39, wherein each respective dormant patient display includes: the video imagery received from each warming therapy device; and the button by which a caregiver may selectively play the neonatal patient's vocalizations.
Clause 41. The neonatal patient monitoring system of clause 39, wherein at least one of the warming therapy devices is an incubator.
Clause 42. The neonatal patient monitoring system of clause 39, wherein the plurality of sensors for each warming therapy device includes: a camera by which the video imagery is captured, the camera positioned in the care environment and directed at the neonatal patient location; and a microphone positioned in the care environment by which the neonatal patient's vocalizations are captured.
Clause 43. The neonatal patient monitoring system of clause 42, wherein the camera and the microphone are integrated into a single device.
Clause 44. The neonatal patient monitoring system of clause 42, wherein the camera and the microphone are integrated into the respective warming therapy apparatus.
Clause 45. The neonatal patient monitoring system of clause 39, wherein the plurality of sensors includes: one or more environmental condition sensors to sense environmental parameters of the care environment; one or more patient condition sensors to sense patient condition parameters; and one or more warming therapy device condition sensors to sense operational state of the warming therapy device.
Clause 46. The neonatal patient monitoring system of clause 45, wherein the environmental condition sensors sense humidity, or oxygen levels, or air temperature, or a combination thereof.
Clause 47. The neonatal patient monitoring system of clause 45, wherein the patient condition sensors sense blood oxygen saturation, or body temperature, or skin temperature, or skin galvanic response, or patient motion, or blood pressure, or a combination thereof.
Clause 48. The neonatal patient monitoring system of clause 45, wherein the warming therapy device condition sensors sense power to the warming therapy device, warming therapy device on, warming therapy device closed, or door latch status, or warming therapy device setting, or combinations thereof.
Clause 49. The neonatal patient monitoring system of clause 39, wherein the central monitoring controller is further programmed to, in the first state, provide a plurality of soft key controls by which a caregiver may selectively control information presented on the display device.
Clause 50. The neonatal patient monitoring system of clause 39, wherein the central monitoring controller is further programmed to, in the second state, provide a plurality of soft key controls in the active patient display by which a caregiver may selectively control information presented on the display device.
Clause 51. The neonatal patient monitoring system of clause 39, wherein the central monitoring controller is further programmed to, in the first state, provide selected received patient care data in each respective dormant patient display.
Clause 52. The neonatal patient monitoring system of clause 39, wherein the central monitoring controller is further programmed to, in a third state, selectively provide the active patient display on the display device upon selection of the particular warming therapy device by the caregiver.
Clause 53. A method for neonatal monitoring, comprising: for each of a plurality of warming therapy devices, sensing a plurality of patient care data from within a care environment for a neonatal patient, the patient care data including: video imagery of the neonatal patient; and audio capture of vocalizations of the neonatal patient; and receiving the sensed patient care data at a warming therapy device, the warming therapy device programmed to: transmit the received patient care data; analyze the received patient care data for alarm conditions; and if an alarm condition is detected, transmit an alarm signal; and receiving at a centralized monitoring station the transmitted patient care data and the transmitted alarm signals from each warming therapy device, the centralized transmitting station: in a first state, concurrently provide a respective dormant patient display for each warming therapy device on the display device; and in a second state, automatically provide on the display device an active patient display for a particular warming therapy device triggered by receiving an alarm signal transmitted from the particular warming therapy device, the active patient display being larger than the dormant patient display and including: the video imagery received from each warming therapy device; a button by which a caregiver may selectively play the neonatal patient's vocalizations; an identity of the alarm condition; and received patient care data associated with the alarm condition.
Clause 54. The method of clause 53, wherein each respective dormant patient display includes: the video imagery received from each warming therapy device; and the button by which a caregiver may selectively play the neonatal patient's vocalizations.
Clause 55. The method of clause 53, wherein the plurality of sensors for each warming therapy device includes: a camera by which the video imagery is captured, the camera positioned in the care environment and directed at the neonatal patient location; and a microphone positioned in the care environment by which the neonatal patient's vocalizations are captured.
Clause 56. The method of clause 53, wherein the plurality of patient care data includes: one or more environmental parameters of the care environment; one or more patient condition parameters; and one or more operational states of the warming therapy device.
Clause 57. The method of clause 53, wherein the environmental parameters include humidity, or oxygen levels, or air temperature, or a combination thereof.
Clause 58. The method of clause 53, wherein the patient condition parameters include blood oxygen saturation, or body temperature, or skin temperature, or skin galvanic response, or patient motion, or blood pressure, or a combination thereof.
Clause 59. The method of clause 53, wherein the warming therapy device condition parameters include power to the warming therapy device, warming therapy device on, warming therapy device closed, or door latch status, or warming therapy device setting, or combinations thereof.
Clause 60. The method of clause 53, wherein, in the first state, the dormant patient display includes a plurality of soft key controls by which a caregiver may selectively control information presented on the display device.
Clause 61. The method of clause 53, wherein, in the second state, the active patient display includes a plurality of soft key controls in the active patient display by which a caregiver may selectively control information presented on the display device.
Clause 62. The method of clause 53, wherein, in the first state, the dormant patient display includes selected received patient care data in each respective dormant patient display.
Clause 63. The method of clause 53, wherein the central monitoring controller is further programmed to, in a third state, selectively provide the active patient display on the display device upon selection of the particular warming therapy device by the caregiver.
Clause 64. A neonatal patient monitoring system substantially as shown and described.
Clause 65. A method for neonatal monitoring substantially as shown and described.

## Claims

1. A neonatal patient monitoring system, comprising:
a central monitoring station, including:
a display device, and
a central monitoring controller programmed to:
receive a plurality of transmitted patient care data from each of a plurality of warming therapy devices;
in a first state, concurrently provide a respective dormant patient display for each warming therapy device on the display device; and
in a second state, provide on the display device an active patient display for a particular one of the warming therapy devices, the active patient display being larger than the dormant patient display.

2. The neonatal patient monitoring system of claim 1, wherein the active patient display is automatically provided upon receipt of an alarm signal from the particular one of the warming therapy devices, optionally wherein the active patient display includes:
video imagery received from the particular warming therapy device;
a button by which a caregiver may selectively play the neonatal patient's vocalizations;
an identity of the alarm condition; and
received patient care data associated with the alarm condition.

3. The neonatal patient monitoring system of claim 1 or 2, wherein the active patient display is provided upon selection of a caregiver.

4. The neonatal patient monitoring system of any of the preceding claims, wherein the active patient display includes:
video imagery received from the particular warming therapy device;
a button by which a caregiver may selectively play the neonatal patient's vocalizations; and
received patient care data selected by the caregiver.

5. The neonatal patient monitoring system of any of the preceding claims, wherein the patient care data includes:
video imagery received from the particular warming therapy device; and
audio data of the neonatal patient's vocalizations which a caregiver may selectively play with a button of the active patient display; and
a skin galvanic response of the neonatal patient.

6. The neonatal patient monitoring system of any of the preceding claims, further comprising:
the plurality of warming therapy devices, each warming therapy device defining a respective care environment and including:
a plurality of sensors to capture patient care data when the warming therapy device is in use, the plurality of sensors including:
a camera positioned in the respective care environment and directed on a patient location to capture video imagery of the neonatal patient;
a microphone positioned in the care environment to capture
vocalizations of the neonatal patient; and optionally a galvanic skin response sensor to capture a galvanic electrodermal
response of the neonatal patient;
a warming therapy device controller programmed to:
transmit the captured patient care data; and optionally:
analyze the captured patient care data for alarm conditions; and
if an alarm condition is detected, transmit an alarm signal.

7. The neonatal patient monitoring system of claim 6, wherein:
the camera and the microphone are integrated into a single device; and/or
the camera and the microphone are integrated into the respective warming therapy apparatus; and
optionally wherein at least one of the warming therapy devices of the plurality of warming therapy devices is an incubator.

8. The neonatal patient monitoring system of claim 6 or 7, wherein each warming therapy device includes:
a plurality of sensors, including:
one or more environmental condition sensors to sense environmental parameters of the care environment, optionally wherein the environmental condition sensors sense humidity, oxygen levels, or air temperature, or a combination thereof;
one or more patient condition sensors to sense patient condition parameters, optionally wherein the patient condition sensors sense blood oxygen saturation, blood pressure, end-tidal carbon dioxide, or body temperature, or skin temperature, or skin galvanic response, or motion, or a combination thereof; and
one or more warming therapy device condition sensors to sense operational state of the warming therapy device, optionally wherein the warming therapy device condition sensors sense power to the warming therapy device, warming therapy device on, warming therapy device closed, or door latch status, or warming therapy device setting, or combinations thereof.

9. The neonatal patient monitoring system of any of claims 2-8, wherein each respective dormant patient display includes:
the video imagery received from each warming therapy device; and
the button by which a caregiver may selectively play the neonatal patient's vocalizations.

10. The neonatal patient monitoring system of any of the preceding claims, wherein the central monitoring controller is further programmed to:
in the first state:
provide a plurality of soft key controls by which a caregiver may selectively control information presented on the display device; and/or
provide selected received patient care data in each respective dormant patient display; and/or
in the second state:
provide a plurality of soft key controls in the active patient display by which a caregiver may selectively control information presented on the display device; and/or
in a third state:
selectively provide the active patient display on the display device upon selection of the particular warming therapy device by the caregiver.

11. A method for neonatal monitoring, comprising:
receiving a plurality of transmitted patient care data from each of a plurality of warming therapy devices;
in a first state, concurrently providing a respective dormant patient display for each warming therapy device on the display device; and
in a second state, providing on the display device an active patient display for a particular one of the warming therapy devices, the active patient display being larger than the dormant patient display.

12. The method of claim 11, wherein providing the active patient display includes automatically providing upon receipt of an alarm signal from the particular one of the warming therapy devices, optionally wherein providing the active patient display includes providing:
video imagery received from the particular warming therapy device;
a button by which a caregiver may selectively play the neonatal patient's vocalizations;
an identity of the alarm condition; and
received patient care data associated with the alarm condition.

13. The method of claim 11, wherein providing the active patient display includes:
providing the active patient display upon selection of a caregiver; and optionally further includes providing:
video imagery received from the particular warming therapy device;
a button by which a caregiver may selectively play the neonatal patient's vocalizations; and
received patient care data selected by the caregiver;
optionally wherein each respective dormant patient display includes:
the video imagery received from each warming therapy device; and
the button by which the caregiver may selectively play the neonatal patient's vocalizations

14. The method of claim 11, further comprising:
sensing the plurality of patient care data by each of the plurality of warming therapy devices, including:
acquiring the video imagery from within the care environment;
capturing the neonatal patient's vocalizations from within the care environment; and optionally
sensing a galvanic skin response of the neonatal patient; and transmitting the patient care data from the warming therapy devices, optionally wherein the method further comprises by each respective warming therapy device:
analyzing the captured patient data for alarm conditions; and
if an alarm condition is detected, transmitting an alarm signal.

15. The method of any of claims 11-14, wherein the patient care data includes:
one or more environmental parameters of the care environment, optionally wherein the environmental parameters include humidity, oxygen levels, or air temperature, or a combination thereof;
one or more patient condition parameters, optionally wherein the patient condition sensors sense blood oxygen saturation, blood pressure, end-tidal carbon dioxide, or body temperature, or skin temperature, or skin galvanic response, or motion, or a combination thereof; and
one or more operational states of the warming therapy device, optionally wherein the warming therapy device condition sensors sense power to the warming therapy device, warming therapy device on, warming therapy device closed, or door latch status, or warming therapy device setting, or combinations thereof.
